# FASCICULE DE BREVET EUROPEEN

(11) **EP 1 765 366 B1**
(45) Date de publication et mention de la délivrance du brevet: **11.01.2012**
(21) Numéro de dépôt: 05775263.6
(22) Date de dépôt: 06.06.2005
(51) Int. Cl.: A61K 31/737, A61P 19/02, A61P 17/02, A61L 15/28

(54) **UTILISATION DE DERIVES DEPOLYMERISES SULFATES D'EXOPOLYSACCHARIDES (EPS), COMME CICATRISANTS**
WUNDHEILENDE VERWENDUNG VON SULFATIERTEN DEPOLYMERISIERTEN EXOPOLYSACCHARIDDERIVATE
USE OF SULPHATED DEPOLYMERISED DERIVATES OF EXOPOLYSACCHARIDES (EPS) AS WOUND HEALING AGENTS

(30) Priorité: 14.06.2004 FR 0406405
(43) Date de publication de la demande: 28.03.2007
(73) Titulaire: Institut Français de Recherche pour l'Exploitation de la Mer (IFREMER), 92138 Issy-les-Moulineaux (FR); Université René Descartes - Paris 5, 75270 Paris Cedex 06 (FR)
(72) Inventeur: SENNI, Karim, F-93600 Aulnay sous Bois (FR); GEUNICHE, Farida, F-92500 Rueil Malmaison (FR); YOUSFI, Miriam, F-75014 Paris (FR); FIORETTI, Florence, F-75014 Paris (FR); GODEAU, Gaston-Jacques, F-92160 Antony (FR); COLLIEC-JOUAULT, Sylvia, F-44100 Nantes (FR); RATISKOL, Jacqueline, F-44980 Sainte Luce sur Loire (FR); SINQUIN, Corinne, F-44300 Nantes (FR); RAGUENES, Gérard, F-29280 Locmaria Plouzane (FR); COURTOIS, Anthony, F-29290 Saint Renan (FR); GUEZENNEC, Jean, F-29280 Plouzane (FR)
(74) Mandataire: Cabinet Plasseraud
(86) Numéro de dépôt international: PCT/FR2005/001379
(87) Numéro de publication internationale: WO 2006/003290

(56) Documents cités:
- WO-A-01/15654
- WO-A-99/67411
- WO-A-03/106506
- FR-A- 2 701 488
- FR-A- 2 755 142
- PATENT ABSTRACTS OF JAPAN vol. 1998, no. 13, 30 novembre 1998 (1998-11-30) -& JP 10 218902 A (SEIKAGAKU KOGYO CO LTD), 18 août 1998 (1998-08-18)
- COLLIEC JOUAULT A S ET AL: "Characterization, chemical modifications and in vitro anticoagulant properties of an exopolysaccharide produced by Alteromonas infernus" BBA - GENERAL SUBJECTS, ELSEVIER SCIENCE PUBLISHERS, NL, vol. 1528, no. 2-3, 3 octobre 2001 (2001-10-03), pages 141-151, XP004312011 ISSN: 0304-4165
- GUEZENNEC J ET AL: "Sulfation and depolymerization of a bacterial exopolysaccharide of hydrothermal origin" CARBOHYDRATE POLYMERS, APPLIED SCIENCE PUBLISHERS, LTD. BARKING, GB, vol. 37, no. 1, septembre 1998 (1998-09), pages 19-24, XP004141130 ISSN: 0144-8617
- ZUBKOV V A ET AL: "Structure of the capsular polysaccharide from Alteromonas sp. CMM 155" CARBOHYDRATE RESEARCH, ELSEVIER SCIENTIFIC PUBLISHING COMPANY. AMSTERDAM, NL, vol. 275, no. 1, 15 septembre 1995 (1995-09-15), pages 147-154, XP004021934 ISSN: 0008-6215

## Description

La présente invention concerne des dérivés polysaccharidiques obtenus à partir de polysaccharides natifs d'un genre particulier, appelés exopolysaccharides (EPS), qui sont excrétés par diverses souches de bactéries marines mésophiles issues du milieu hydrothermal profond.

Plus particulièrement, la présente invention concerne des dérivés polysaccharidiques de faible masse molaire et hautement sulfatés, susceptibles d'être obtenus par dépolymérisation radicalaire et sulfatation des exopolysaccharides.

On connaît déjà des polysaccharides hautement sulfatés et de faible masse molaire qui ne sont pas des dérivés d'exopolysaccharides bactériens. Ils présentent des propriétés intéressantes, notamment en tant que substances thérapeutiques. Par exemple, l'héparine, polysaccharide sulfaté, est l'agent anticoagulant et antithrombotique le plus utilisé dans la prévention et le traitement de la thrombose veineuse. Les héparines commercialisées sont actuellement extraites des muqueuses intestinales de porc. Toutefois, l'utilisation de ces héparines d'origine animale présente un risque de contamination par des agents pathogènes (par exemple le prion). Afin de réduire ou d'éviter le risque de contamination, l'identification de nouveaux polysaccharides provenant d'origines autres qu'animales apparaît comme une voie de recherche particulièrement prometteuse.

Les polysaccharides provenant des bactéries, et plus particulièrement des bactéries marines, permettent de répondre à ce besoin. L'étude et l'exploitation des polysaccharides excrétés par les bactéries marines ou exopolysaccharides s'inscrit donc dans cet axe de recherche, notamment pour la conception de nouveaux principes actifs ou d'analogues de molécules déjà existantes.

Un criblage d'échantillons issus du milieu hydrothermal des profondeurs des océans a permis d'isoler une grande variété de souches bactériennes mésophiles, capables de produire des EPS natifs à pression atmosphérique et à température ambiante.

Un petit nombre d'EPS natifs a déjà fait l'objet de dépôts de brevets et de publications : par exemple, le brevet européen EP 975 791 décrit la souche *Vibrio diabolicus,* l'EPS HE 800 natif et son utilisation en tant que médicament, dont notamment comme agent rétroviral, antitumorale et antithrombotique. A l'état natif, l'EPS HE 800 de 800 000 g/mol n'est pas sulfaté ; il est constitué approximativement de 30 % en poids d'osamine, 32 % en poids d'oses acides et 1 % en poids d'oses neutres ; sa teneur en protéines est proche de 1% en poids. La demande de brevet européen N° 1 296 695 décrit l'utilisation de l'EPS HE 800 sous sa forme native comme matériau facilitant la cicatrisation osseuse (Zanchetta et al, Calcif Tissue Int, 2003, 72 :74-79).

Un deuxième EPS natif dénommé GY 785 et produit par la bactérie *Alteromonas infernus* a également été identifié et décrit dans le brevet français N° 2 755 142. L'EPS GY 785 natif est constitué d'une population hétérogène de chaînes polysaccharidiques de masse molaire moyenne supérieure à 10⁶ g/mol. L'EPS natif GY 785 est faiblement sulfaté (taux de sulfate inférieur à 10 % en poids) ; il est constitué de 57 % en poids d'oses neutres (glucose et galactose majoritairement) et 42 % en poids d'oses acides (acide glucuronique et acide galacturonique) ; il ne comprend pas d'osamines et de substituants acétate, lactate, pyruvate et succinates ; sa teneur en protéine est environ de 4 % en poids (Guezennec J., Ind. Microb. Biotech., 2002, 29 : 204-208).

Un troisième EPS natif dénommé ST 716 et produit par la bactérie *Alteromonas macleodii subsp. fijiensis* a également été identifié et décrit dans le brevet européen EP 1171625 (Rougeaux H. et al., Carbohydr. Res., 1998, 312 : 53-59). L'EPS ST 716 natif est faiblement sulfaté (son taux de sulfate est approximativement de 5 % en poids) ; il est constitué de 40% en poids d'oses neutres et 40% d'oses acides ; sa teneur en protéines est de 2 à 4% en poids.

D'autres EPS natifs nouveaux ont également été identifiés et décrits dans la littérature ; notamment l'EPS HYD 721 produit par une *Pseudoalteromonnas* (Rougeaux H. et al., Carbohydr Res., 1999, 315 : 273-285 et Raguenes G. et al., 1997), l'EPS HYD 657 (Cambon-Bonavita M. et al., J Applied Microbiol, 2002, 93 : 310-315) et l'EPS MS 907 (Raguenes G. et al., Curr Microbiol, 2003, 46 : 448-52).

La présente invention a pour objet des dérivés polysaccharidiques sulfatés provenant du traitement d'EPS natifs excrétés par des souches bactériennes mésophiles issues du milieu hydrothermal profond et ayant un intérêt pharmaceutique, cosmétique ou en ingénierie tissulaire pour utilisation comme agents cicatrisants. Les dérivés polysaccharidiques sulfatés d'exopolysaccharides (EPS) natifs excrétés par des bactéries marines mésophiles issues du milieu hydrothermal profond selon l'invention sont susceptibles d'être obtenus par le procédé comprenant les étapes suivantes :
- une étape de dépolymérisation radicalaire desdits EPS natifs, pour l'obtention de dérivés dépolymérisés de faible masse molaire, inférieure ou égale à 100000 g/mol,
- une étape ultérieure de sulfatation des dérivés dépolymérisés, éventuellement lyophilisés, comprenant l'addition d'au moins un agent de sulfatation en une quantité suffisante pour obtenir des dérivés polysaccharidiques sulfatés présentant un taux de substitution en groupements sulfates compris entre 10 et 45 % en poids par rapport au poids total du dérivé polysaccharidique sulfaté, ladite étape de sulfatation étant éventuellement suivie d'une étape de dialyse.

Lors de la première étape de dépolymérisation, l'EPS natif peut être utilisé sous une forme liquide c'est-à-dire tel qu'il est excrété par les bactéries dans le milieu de culture. Préférentiellement, le milieu de culture est centrifugé, et seul le surnageant contenant l'EPS natif et dépourvu de débris bactériens est conservé. L'EPS natif peut être recueilli par toute technique appropriée bien connue de l'homme du métier, notamment par ultrafiltration sur membrane, puis éventuellement être lyophilisé tel quel ou sous la forme d'un sel d'addition.

L'étape de dépolymérisation par voie radicalaire de l'EPS natif est de préférence réalisée par addition d'une solution d'un agent oxydant à un mélange réactionnel comprenant l'EPS natif, de préférence en présence d'un catalyseur métallique. L'agent oxydant est de préférence choisi parmi les peroxydes, notamment le peroxyde d'hydrogène, et les peracides, notamment l'acide peracétique et l'acide 3-chloro-perbenzoïque. L'addition est de préférence effectuée en continu et sous agitation pendant une durée comprise entre 30 minutes et 10 heures. Le mélange réactionnel est de préférence maintenu à un pH compris entre 6 et 8, par exemple par ajout continu d'un agent alcalinisant tel que la soude, et à une température comprise entre 30 et 70°C environ pendant toute la durée de la réaction de dépolymérisation radicalaire.

Selon un mode de réalisation particulier de la présente invention, lors de cette étape, l'EPS natif est présent dans le mélange réactionnel à une concentration comprise entre environ 2 et 10 mg/ml de mélange réactionnel.

Selon un mode de réalisation préféré de l'invention, l'agent oxydant est une solution de peroxyde d'hydrogène (H₂O₂) ayant de préférence une concentration comprise entre 0,1 % et 0,5 % en poids environ, de préférence de l'ordre de 0,1 à 0,2 % en poids, qui est ajoutée à un débit de V1/1000 à V1/10 ml/minute, de préférence V1/50 et V1/500 ml/minutes, très préférentiellement de l'ordre de V1/100 ml/minutes, V1 étant le volume du milieu réactionnel contenant un exopolysaccharide (EPS) marin auquel est ajouté une solution de peroxyde d'hydrogène.

Les catalyseurs métalliques utilisables lors de l'étape de dépolymérisation sont de préférence choisis parmi les ions Cu⁺⁺, Fe ⁺⁺, Cr ⁺⁺⁺ et l'anion Cr₂O₇²⁻ tels que décrits notamment dans la demande de brevet EP 0 221 977. Selon un mode de réalisation particulier, le catalyseur métallique est présent dans le mélange réactionnel à une concentration comprise entre 10⁻³ M et 10⁻¹ M environ, et préférentiellement à une concentration comprise entre 0,001 et 0,05 M environ.

Le procédé de dépolymérisation radicalaire conforme à l'invention et tel que décrit ci-dessus permet d'obtenir, en une seule étape, sans fractionnement par chromatographie d'exclusion stérique, et avec un bon rendement, des dérivés polysaccharidiques homogènes de faible masse molaire. Par « dérivés polysaccharidiques de faible masse molaire » on entend des dérivés de masse molaire inférieure ou égale à 100 000 g/mol, de préférence comprise entre 5 000 et 50 000 g/mol, et plus préférentiellement inférieure ou égale à 25 000 g/mol. Dans le cadre de l'exposé de la présente invention, on entend par "dérivés homogènes", des dérivés qui, en chromatographie d'exclusion stérique haute performance, présentent un seul pic principal représentant une population majoritaire de chaînes polysaccharidiques homogènes en taille caractérisée par un indice de polydispersité I (Mp/Mn) <5, de préférence compris entre 1.5 et 4, plus préférentiellement inférieur ou égale à 2 avec Mp = masse molaire moyenne en poids et Mn = masse molaire moyenne en nombre.

Lorsque la réaction de dépolymérisation est terminée, suivant un mode de réalisation particulier de l'invention, le procédé comprend une étape de réduction des dérivés polysaccharidiques obtenus, à l'aide d'un agent réducteur, de façon à stabiliser les chaînes dont les extrémités réductrices sont très réactives et notamment pour éviter une hydrolyse des chaînes par la réaction dite de "peeling". La nature des agents réducteurs utilisables à cet effet n'est pas critique. Il peut notamment s'agir du borohydrure de sodium.

Le catalyseur métallique utilisé pour la dépolymérisation peut être éliminé à l'issue de la réaction de dépolymérisation, et dans le mode de réalisation dans lequel une étape de réduction est effectuée, à l'issue de la réduction, par chromatographie d'échange d'ions, de préférence une résine échangeuse faible de cations préalablement passivée, ou par traitement à l'EDTA (Ethylène Diamine Tétra-Acétique).

Selon un mode de réalisation particulier du procédé de l'invention, préalablement à l'étape de sulfatation, il est procédé à une étape de N-désacétylation des dérivés polysaccharidiques comprenant des hexosamines N-acétylées et obtenus à l'issue de l'étape de dépolymérisation radicalaire et/ou à l'issue de l'étape de réduction. Cette étape de N-désacétylation est réalisée selon un protocole adapté de Zou et al (Carbohyd. Res., 1998, 309 : 297-301). Avantageusement, l'étape de N-désacétylation est réalisée par addition au mélange réactionnel comprenant les dérivés polysaccharidiques, d'une solution de borohydrure de sodium, sous agitation. Lorsque la température du mélange réactionnel atteint approximativement 80°C, un agent alcalinisant, préférentiellement la soude est ajouté au milieu réactionnel. Le mécanisme de l'hydrolyse basique d'un amide en milieu basique, et préférentiellement en présence de soude est schématisé ci-dessous :

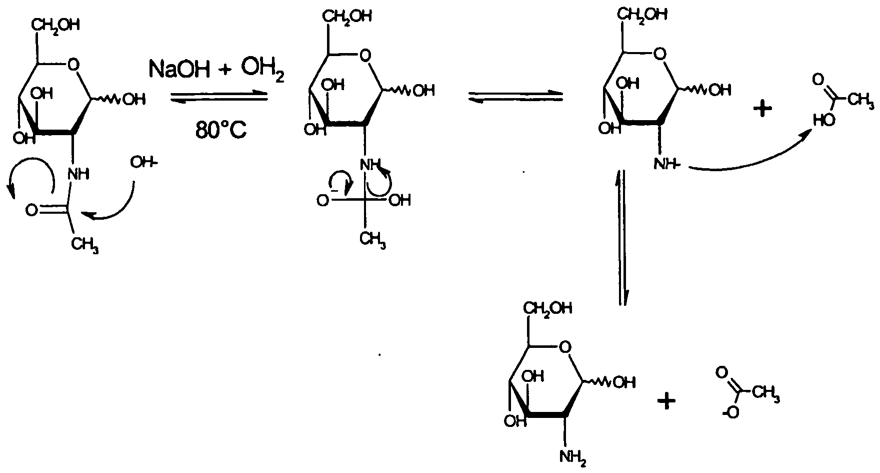

Après une heure de réaction, le milieu réactionnel est neutralisé par ajout continu d'acide acétique jusqu'à l'obtention d'un pH de 5. Les dérivés polysaccharidiques obtenus peuvent être recueillis par ultrafiltration sur membrane, puis éventuellement être lyophilisés.

Selon un mode de réalisation préférentiel, l'étape de N-désacétylation est mise en oeuvre sur les dérivés polysaccharidiques provenant de la dépolymérisation d'EPS natifs excrétés par des bactéries marines mésophiles hydrothermales du genre *Vibrio,* de préférence HE 800. Les EPS natifs excrétés par les dites bactéries du genre *Vibrio* sont caractérisés par le fait qu'ils renferment des hexosamines N-acétylées.

Les dérivés polysaccharidiques résultant de la dépolymérisation, et/ou de la réduction et/ou de la N-désacétylation peuvent, si nécessaire, être recueillis par toute technique appropriée bien connue de l'homme du métier telle que par exemple par ultrafiltration sur membrane, puis éventuellement être lyophilisés tels quels ou sous la forme d'un sel d'addition avec une base faible ou forte, pouvant par exemple être choisie parmi la pyridine, la triéthylamine, la tributylamine, l'hydroxyde de tétrabutylammonium et la soude. Ce sel lyophilisé peut par exemple être préparé par élution d'une solution aqueuse des dérivés polysaccharidiques à une concentration comprise entre 1 et 8 mg/ml sur une colonne de résine échangeuse d'ions telle que par exemple celles vendues sous la dénomination Dowex ® par la société Dow Chemical. L'éluat est collecté tant que le pH reste acide, par exemple inférieur à 5 puis le pH est ensuite ajusté à environ 6,5 avec la base désirée telle que définie ci-dessus. Les dérivés polysaccharidiques sous forme d'un sel sont alors ultrafiltrés et lyophilisés.

Les dérivés polysaccharidiques lyophilisés, sous forme de sel d'addition ou non, sont de préférence dissous dans un solvant anhydre au début de l'étape de sulfatation ; ce solvant est de préférence choisi parmi le diméthylformamide (DMF), le diméthylsulfoxyde (DMSO) et/ou le formamide. La quantité de dérivés polysaccharidiques présente au sein du solvant anhydre peut être comprise entre 1 et 10 mg/ml environ, de préférence entre 1 et 5 mg/ml environ et encore plus préférentiellement cette quantité est de 2,5 mg/ml environ. La dissolution de l'EPS dans le solvant anhydre est de préférence réalisée, sous agitation, à température ambiante pendant 1 à 2 heures environ puis à une température comprise entre 40 et 50 °C, de préférence à une température d'environ 45°C pendant environ 2 heures sous argon avec du tamis moléculaire.

Le ou les agents de sulfatation chimique utilisés lors de l'étape de sulfatation peuvent être additionnés aux EPS dépolymérisés et/ou réduits et/ou N-désacétylés qui sont sous forme lyophilisée ou sous la forme d'une solution.

Les agents de sulfatation sont de préférence choisis parmi les complexes de pyridine sulfate (libre ou couplée à un polymère), de diméthylformamide sulfate, triéthylamine sulfate, et de triméthylamine sulfate. Le ou les agents de sulfatation chimique sont ajoutés à la solution de dérivés polysaccharidiques en une quantité pondérale représentant de préférence de 4 à 6 fois environ et encore plus préférentiellement 5 fois environ la masse de dérivés polysaccharidiques en solution. La réaction de sulfatation chimique est alors de préférence conduite sous agitation pendant une durée comprise entre 2 et 24 heures environ selon le degré de sulfatation souhaité. Lorsque le degré de sulfatation désiré est atteint, la réaction de sulfatation est arrêtée après refroidissement du milieu réactionnel :
- soit par addition d'eau dans une proportion de préférence égale à 1/10 du volume réactionnel et ajustement du pH du milieu réactionnel à 9 avec un agent alcalinisant tel que par exemple la soude (3 M);
- soit et de préférence, par précipitation en présence d'acétone saturée en chlorure de sodium ou de méthanol puis dissolution du précipité dans l'eau.

Selon une mode de réalisation particulier, la solution de dérivés polysaccharidiques sulfatés est de préférence dialysée afin éliminer les différents sels puis lyophilisée.

Dans le contexte de l'invention, par « dérivés polysaccharidiques sulfatés », on entend des dérivés polysaccharidiques ayant été soumis à un traitement de sulfatation chimique et comprenant des groupement sulfates, qu'ils aient ou n'aient pas de groupement sulfates avant ce traitement de sulfatation.

Préférentiellement, les dérivés polysaccharidiques de faible masse molaire et sulfatés conformes à l'invention ont une masse molaire inférieure ou égale à 100 000 g/mol, de préférence compris entre 5 000 et 50 000 g/mol, un indice de polydispersité inférieur à 5, de préférence compris entre 1,5 et 4 et un taux de substitution en groupements sulfates compris entre 10 et 45 % en poids, et de préférence compris entre 20 et 40 % en poids inclusivement.

Plus préférentiellement, les dérivés polysaccharidiques de faible masse molaire et sulfatés conformes à l'invention ont une masse molaire inférieure ou égale à 25 000 g/mol, un indice de polydispersité inférieur à 2, et un taux de substitution en groupements sulfates compris entre 10 et 45 % en poids, et de préférence compris entre 20 et 40 % en poids inclusivement.

Les dérivés polysaccharidiques de faible masse molaire et sulfatés conformes à l'invention sont obtenus par traitement d'EPS natifs excrétés par des bactéries marines mésophiles d'origine hydrothermale appartenant de préférence au genre *Alteromonas* ou *Vibrio.*

Selon une variante de l'invention, les bactéries du genre *Alteromonas* sont sélectionnées parmi les souches GY 785, HYD 657, HYD 721, HYD 1545, HYD 1644, ST 716 et MS 907.

L'invention concerne des dérivés polysaccharidiques de faible masse molaire et sulfatés obtenus à partir d'EPS natifs excrétés par des bactéries du genre *Alteromonas,* lesdits EPS natifs ayant une teneur de 20% à 70%, préférentiellement de 30% à 60%, et plus préférentiellement de 38% à 57% en poids d'oses neutres.

L'invention concerne également des dérivés polysaccharidiques de faible masse molaire et sulfatés obtenus à partir d'EPS natifs excrétés par des bactéries du genre *Alteromonas,* lesdits EPS natifs ayant une teneur de 5% à 60%, préférentiellement entre 6% et 50%, et plus préférentiellement de entre 8% et 42% en poids d'oses acides.

L'invention concerne également des dérivés polysaccharidiques de faible masse molaire et sulfatés obtenus à partir d'EPS natifs excrétés par des bactéries du genre *Alteromonas,* lesdits EPS natifs ayant une teneur de 0 à 1 % en poids d'osamines dans leur composition osidique.

Selon un mode de réalisation particulier, les dérivés polysaccharidiques de faible masse molaire et sulfatés de l'invention sont obtenus à partir d'EPS natifs excrétés des bactéries du genre *Alteromonas,* lesdits EPS natifs ont une composition osidique comprenant :
- de 20% à 70%, préférentiellement de 30% à 60%, et plus préférentiellement de 38% à 57% en poids d'oses neutres,
- de 5% à 60%, préférentiellement de 6% à 50%, et plus préférentiellement de 8% à 42% en poids d'oses acides,
- de 0 à 1 % en poids d'osamines.

Selon un autre mode de réalisation particulier, les dérivés polysaccharidiques de faible masse molaire et sulfatés de l'invention sont obtenus à partir d'EPS natifs excrétés des bactéries du genre *Vibrio,* de préférence par la souche bactérienne HE 800. Les EPS natifs excrétés des bactéries du genre *Vibrio* ne sont pas sulfatés.

L'invention concerne des dérivés polysaccharidiques de faible masse molaire et sulfatés obtenus à partir d'EPS natifs excrétés par des bactéries du genre *Vibrio,* lesdits EPS natifs ayant une teneur de 0% à 5%, préférentiellement de 0% à 1% en poids d'oses neutres.

L'invention concerne des dérivés polysaccharidiques de faible masse molaire et sulfatés obtenus à partir d'EPS natifs excrétés par des bactéries du genre *Vibrio,* lesdits EPS natifs ayant une teneur de 20% à 50%, préférentiellement de 25% à 40%, et plus préférentiellement de 30% à 32% en poids d'oses acides.

L'invention concerne des dérivés polysaccharidiques de faible masse molaire et sulfatés obtenus à partir d'EPS natifs excrétés par des bactéries du genre *Vibrio,* lesdits EPS natifs ayant une teneur de 20% à 50%, préférentiellement de 25% à 40%, et plus préférentiellement de 30% à 35% en poids d'osamines.

L'invention concerne des dérivés polysaccharidiques de faible masse molaire et sulfatés obtenus à partir d'EPS natifs excrétés par des bactéries du genre *Vibrio,* lesdits EPS natifs ayant une teneur de 0 à 15 %, préférentiellement de 4 à 8%, et plus préférentiellement de 5 à 6 % en poids de groupements N-acétylés.

Selon un mode particulier de réalisation de l'invention, les dérivés polysaccharidiques de faible masse molaire et sulfatés conformes à l'invention sont caractérisés par le fait qu'ils sont obtenus à partir d'EPS natifs excrétés par des bactéries du genre *Vibrio,* lesdits EPS natifs ont une composition osidique comprenant :
- de 0 à 5 %, préférentiellement de 0% à 1%, en poids d'oses neutres,
- de 20% à 50%, préférentiellement de 25% à 40%, et plus préférentiellement de 30% à 32% en poids d'oses acides,
- de de 20% à 50%, préférentiellement de 25% à 40%, et plus préférentiellement de 30% à 35% en poids d'osamines,
- de 0% à 15%, préférentiellement 4 à 8%, et plus préférentiellement de 5 à 6 % en poids de groupements N-acétylés.

L'invention concerne des dérivés polysaccharidiques de faible masse molaire et sulfatés obtenus à partir d'EPS natifs qui ont une teneur de 0 à 15%, préférentiellement de 0 à 5 %, et plus préférentiellement de 0 à 1% en poids de protéines.

De façon surprenante et inattendue, les inventeurs ont mis en évidence que les dérivés polysaccharidiques de faible masse molaire et sulfatés conformes à l'invention sont utiles comme agents cicatrisants des tissus conjonctifs, et sont notamment capables de stimuler la prolifération des fibroblastes, d'inhiber la sécrétion des médiateurs solubles ou cytokines pro-inflammatoires par les fibroblastes, d'inhiber la sécrétion de métalloprotéases matricielles par les fibroblastes, d'inhiber la voie classique du complément, de stimuler la prolifération des fibroblastes au détriment des myofibroblastes, de stimuler sélectivement la prolifération des cellules médullaires à vocation mésenchymateuse au détriment d'autres sous populations dans une population hétérogène de cellules.

Les fibroblastes ont un rôle central dans le processus de cicatrisation d'un tissu lésé en vue de la formation d'un tissu de remplacement pour en restaurer sa fonctionnalité. Typiquement, le processus de cicatrisation se déroule en 3 phases au cours desquelles les fibroblastes font progresser les processus de régénération selon des séquences chronologiques précises, mais imbriquées les unes dans les autres :
(1) La première phase de la cicatrisation vasculaire et inflammatoire se caractérise par la libération d'un grand nombre de facteurs de croissance et de cytokines, de protéases et la migration de cellules inflammatoires, fibroblastiques et vasculaires au niveau de la lésion. L'afflux de cellules inflammatoires et la production de cytokines induisent la production d'hydrolases comme les sérines protéases ou les métalloprotéases matricielles par les fibroblastes. Lorsque la phase inflammatoire, normalement transitoire se prolonge sans contrôle, une pathologie inflammatoire chronique s'installe.
(2) La phase de reconstruction (phase proliférative ou tissu de granulation) du tissu se traduit par le comblement de la perte de substances tissulaires survenue lors d'une lésion par une matrice extracellulaire peu organisée et richement vascularisée. Les fibroblastes prolifèrent rapidement sous l'effet de facteurs de croissance. Ces fibroblastes effectuent un travail remarquable de reconstruction en sécrétant des composants de la matrice extracellulaire tels que des glycosaminoglycannes (GAG), la fibronectine et le collagène. Une partie de ces fibroblastes acquiert un phénotype myofibroblastique en exprimant notamment l'α-actine des muscles lisses. Le tissu cicatriciel se rétracte grâce aux capacités contractiles des myofibroblastes.
(3) Lors de la phase de maturation, une grande partie des myofibroblastes disparait par apoptose et est remplacée par des fibroblastes n'exprimant plus l'α-actine des muscles lisses. A ce stade la persistance des myofibroblastes, de part leur activité, peut conduire à des pathologies de type fibrotique. Au terme de ce processus, un tissu fibreux dense cicatriciel s'est constitué et peut alors se remodeler. La maturation du tissu cicatriciel se caractérise notamment par une modification de l'orientation des fibres matricielles qui tendent à se disposer selon les lignes de plus forte tension comme dans un tissu conjonctif normal.

Le remodelage tissulaire est une balance dynamique entre la synthèse de la matrice extracellulaire et sa dégradation. Lorsque cette balance s'équilibre, la cicatrisation est normale. Toutefois, lorsque la balance penche durablement vers la synthèse de matrice extracellulaire, on assiste au développement d'une fibrose. Lorsque la balance penche vers la dégradation excessive de la matrice extracellulaire, une pathologie inflammatoire s'installe.

La présente invention concerne l'utilisation des dérivés polysaccharidiques de faible masse molaire et sulfatés conformes à l'invention comme agents cicatrisants des tissus conjonctifs, notamment des tissus conjonctifs dermiques et gingivaux.

Compte tenu du fait que les dérivés polysaccharidiques de faible masse molaire et sulfatés de l'invention ont des propriétés d'activation de la prolifération des fibroblastes, il est donc particulièrement intéressant de les utiliser en tant qu'agent capable de stimuler la prolifération des fibroblastes, ou en tant qu'agent régénérant pour la préparation d'une composition pharmaceutique à activité cicatrisante, ladite composition permettant notamment de favoriser la reconstruction et le remodelage des tissus conjonctifs, en particulier des tissus conjonctifs dermiques et gingivaux.

Les inventeurs ont également démontré que des dérivés polysaccharidiques de faible masse molaire et sulfatés conformes à l'invention sont utiles comme agents capables d'inhiber la sécrétion des médiateurs solubles ou cytokines pro-inflammatoires par les fibroblastes des tissus conjonctifs, dont notamment l'interleukine 1β (IL-1β) et/ou le TNF-α (Tumor Necrosis Factor).

Certaines pathologies inflammatoires chroniques, tels que les parodontites, les ulcères chroniques, les cicatrisations retardées ou l'arthrite rhumatoïde, s'accompagnent d'une dégradation excessive et incontrôlée des macromolécules matricielles. Ces pathologies sont très souvent associées à la sécrétion délétère de cytokines, notamment de cytokines pro-inflammatoires, à l'activation persistante du complément conduisant, entre autre à la surproduction préjudiciable d'anaphylatoxines chémoattractantes. Dans ces pathologies inflammatoires, la production excessive de cytokines pro-inflammatoires perturbent les fonctions cellulaires et tissulaires physiologiques, dont notamment la migration cellulaire, la prolifération cellulaire, l'expression, la sécrétion et l'activation de certaines protéases comme les métalloprotéases matricielles (MMPs). L'expression de ces MMPs impliquées dans la dégradation des protéines matricielles n'est généralement pas constitutive et est induite par des cytokines pro-inflammatoires telles que l'IL-1β et/ou le TNFα et/ou des facteurs de croissance.

Les inventeurs ont montré que les dérivés polysaccharidiques de faible masse molaire et sulfatés conformes à l'invention sont utiles comme agents capables d'inhiber la sécrétion de métalloprotéases matricielles par les fibroblastes des tissus conjonctifs notamment des tissus conjonctifs dermiques et gingivaux. Notamment, les dérivés selon l'invention sont particulièrement efficaces pour inhiber la sécrétion de la gélatinase A (MMP-2) et/ou de la stromélysine 1 (MMP-3). L'inhibition de la sécrétion de métalloprotéases a été mise en évidence sur des fibroblastes qu'ils soient ou non sous l'influence de cytokines pro-inflammatoires telles que l'IL-1β. Il est en effet particulièrement intéressant de réguler la sécrétion des MMP qui peuvent être responsables de la dégradation incontrôlée des macromolécules matricielles dans des pathologies inflammatoires affectant les tissus conjonctifs dermiques et gingivaux, dont notamment les parodontites ou les ulcérations chroniques.

En régulant la sécrétion de cytokines pro-inflammatoires et de métalloprotéases matricielles des fibroblastes des tissus conjonctifs et en particulier des tissus conjonctifs dermiques et gingivaux, les dérivés sulfatés polysaccharidiques conformes à l'invention montrent une bonne activité anti-inflammatoire.

Le complément est une composante de l'immunité innée (activation spontanée en réponse à une agression) qui se définit comme un ensemble complexe de protéines solubles ou membranaires. Lors d'une réponse inflammatoire, ces protéines s'activent en une série de réactions de protéolyse en chaîne qui engendre des peptides doués d'activités biologiques. L'activation du complément qui est rapide et localisée, est soumise à divers mécanismes de contrôle particulièrement efficaces. Toutefois, certains de ces mécanismes de contrôle sont perturbés dans des pathologies inflammatoires, tel que les pathologies auto-immunes, entraînant une activation persistante du complément. De façon surprenante et inattendue, les inventeurs ont mis en évidence l'utilisation des dérivés polysaccharidiques de faible masse molaire et sulfatés de l'invention comme agents capables d'inhiber la voie classique du complément. Avantageusement, les dérivés polysaccharidiques sulfatés de faible masse molaire et de l'invention sont utilisés comme agents anti-inflammatoires pour la préparation d'une composition pharmaceutique permettant de traiter des pathologies inflammatoires dans lesquelles le complément est activé, notamment des pathologies auto-immunes, comme par exemple les pemphigus.

Compte tenu de leurs propriétés anti-inflammatoires, il est intéressant d'utiliser les dérivés polysaccharidiques sulfatés de faible masse molaire et de l'invention en tant qu'agents anti-inflammatoires pour la préparation d'une composition pharmaceutique permettant notamment de traiter des pathologies inflammatoires, des tissus conjonctifs, notamment des tissus conjonctifs dermiques et gingivaux comme par exemple des parodontites, des ulcérations chroniques ou des cicatrisations retardées.

De nombreuses pathologies sont susceptibles de provoquer l'apparition d'un processus inflammatoire : non seulement les pathologies auto-immunes mais également les pathologies néoplasiques ou infectieuses. Avantageusement, les dérivés polysaccharidiques sulfatés de faible masse molaire et de l'invention sont utilisés en tant qu'agents anti-inflammatoires pour la préparation d'une composition pharmaceutique permettant de traiter des pathologies inflammatoires affectant les tissus conjonctifs dermiques et gingivaux, notamment des pathologies auto-immunes, infectieuses ou néoplasiques, comme par exemple des sarcomes.

Le développement des fibroses pathologiques semble suivre un parcours similaire à celui suivi lors de la régénération tissulaire. Toutefois, le contrôle normal des fonctions cellulaires qui surviennent pendant les processus de régénération tissulaire est perturbé. En effet, des déséquilibres dans la composante cellulaire peuvent se traduire entre autre par l'afflux non contrôlé de cellules inflammatoires entretenant la dégradation tissulaire et/ou par la persistance de sous population cellulaire tels que les myofibroblastes entraînant des pathologies fibrotiques. Alors que les myofibroblastes apparaissent de façon transitoire pendant les processus de cicatrisation normale, cette sous population cellulaire persiste lorsque la régénération tissulaire devient pathologique.

Les inventeurs ont montré que les dérivés polysaccharidiques sulfatés conformes à l'invention sont utiles comme agents capables de stimuler la prolifération des fibroblastes au détriment des myofibroblastes dans les tissus conjonctifs, notamment des tissus conjonctifs dermiques et gingivaux. Plus particulièrement, les dérivés polysaccharidiques sulfatés conformes à l'invention sont utiles comme agents capables de stimuler la prolifération des fibroblastes au détriment des myofibroblastes dans des cultures cellulaires bidimensionnelles ou dans des tissus conjonctifs reconstruits dermiques et gingivaux.

L'utilisation des dérivés polysaccharidiques sulfatés conformes à l'invention permet de stimuler la prolifération des fibroblastes responsables de l'homéostasie tissulaire tout en contrôlant la persistance des myofibroblastes, deux événements cellulaires provenant de la stimulation du processus non pathologique de la régénération tissulaire.

Compte tenu de leur propriété à sélectionner la sous population fibroblastique au détriment de la sous population myofibroblastique, il est donc particulièrement intéressant d'utiliser les dérivés polysaccharidiques de faible masse molaire et sulfatés tels que définis précédemment en tant qu'agent anti-fibrotique pour la préparation d'une composition pharmaceutique, ladite composition permettant notamment de prévenir ou de traiter les processus de cicatrisation hypertrophique ou des pathologies fibrotiques des tissus conjonctifs notamment dermiques et gingivaux.

Cette propriété que les dérivés polysaccharidiques sulfatés conformes à l'invention ont à sélectionner une sous-population cellulaire particulière au sein d'une population hétérogène a également été exploitée pour favoriser l'obtention d'une sous-population de cellules médullaires à vocation mésenchymateuse. Les cellules médullaires à vocation mésenchymateuse sont des cellules souches adultes capables de se développer en cellules mésenchymateuses différenciées selon le tissu dans lequel elles se trouvent, notamment en fibroblastes, chondrocytes, ostéoblastes, adipocytes, et cellules musculaires ayant des caractéristiques morphologiques et des fonctions spécialisées.

Ainsi, les inventeurs ont démontré que les dérivés polysaccharidiques de faible masse molaire et sulfatés conformes à l'invention étaient capables de stimuler sélectivement la prolifération des cellules médullaires à vocation mésenchymateuse au détriment d'autres sous-populations dans une population hétérogène de cellules. Cet effet prolifératif sélectif est d'autant plus intéressant que les cellules médullaires à vocation mésenchymateuse sont rares car particulièrement difficile à obtenir et à maintenir en culture. Il est donc avantageux de complémenter des milieux de culture cellulaire destinés à l'obtention et au maintien des cellules médullaires à vocation mésenchymateuse dans le cadre de thérapie tissulaire ou cellulaire.

Ainsi, les dérivés polysaccharidiques sulfatés conformes à l'invention permettent donc une sélection-amplification par prolifération des cellules médullaires à vocation mésenchymateuse. Ces cellules médullaires à vocation mésenchymateuse représentent une source de cellules pluripotentes utilisables en thérapie tissulaire à des fins de greffe chez l'homme dans la mesure où elles sont capables de se différencier en fibroblastes, en chondrocytes, en ostéoblastes, en adipocytes et en cellules musculaires selon le tissu dans lequel elles sont implantées.

Ces cellules ont donc un intérêt en thérapie tissulaire et cellulaire et plus particulièrement lorsqu'il est impossible de prélever des fragments de peau comme chez les grands brûlés, ou lorsque le tissu ne possède plus les capacités de se régénérer comme le cartilage chez l'adulte.

Chacune des compositions pharmaceutiques ou médicaments contenant les dérivés polysaccharidiques de faible masse molaire et sulfatés obtenus conformément à l'invention peut en outre être utilisée en association avec un ou plusieurs facteurs de croissance présents au sein de la composition pharmaceutique ou présents au sein d'une composition pharmaceutique distincte qui sera alors administrée de façon séparée, c'est-à-dire avant, en même temps ou après l'administration de la composition pharmaceutique renfermant les dérivés polysaccharidiques sulfatés. De tels facteurs de croissance sont en particulier choisis parmi les FGFs (Fibroblast Growth Factors), les TGFβs, les BMPs (Bone Morphogenic Proteins), le CTGF (Connective tissue Growth Factor).

Compte tenu de leurs propriétés sur les fibroblastes, les dérivés polysaccharidiques de faible masse molaire et sulfatés tels que définis précédemment peuvent être utilisés pour la préparation d'une composition pharmaceutique à activité cicatrisante et/ou anti-fibrotique et/ou anti-inflammatoire.

Les compositions pharmaceutiques ou médicaments de l'invention sont destinées à être administrées par toute voie appropriée. La composition pharmaceutique ou le médicament de l'invention se présente préférentiellement sous une forme injectable, dans lequel les dérivés polysaccharidiques sulfatés présentent une masse molaire comprise entre 5 000 et 50 000 g/mol, de préférence inférieure ou égale à 25 000 g/mol, et un indice de polydispersité compris entre 1,5 et 5, de préférence inférieur ou égal à 2 et un taux de substitution en groupements sulfates compris entre 10 et 45 %, et de préférence compris entre 20 et 40 % inclusivement.

La présente invention concerne également une composition cosmétique ou dermatologique pour utilisation comme cicatrisant, caractérisée en ce qu'elle comprend des dérivés polysaccharidiques de faible masse molaire et sulfatés conformes à l'invention en association avec tout excipient approprié.

Préférentiellement, les compositions pharmaceutiques, cosmétiques ou dermatologiques peuvent être administrées localement et se présentées, sous la forme d'un gel, d'une crème, d'une pommade, d'une émulsion, d'une solution.

Elles peuvent également être utilisées *in situ* par l'intermédiaire de substrats, de dispositifs médicaux résorbables ou non, tels que par exemple, des supports à libération différée, des éponges à délitement lent, ou des implants chirurgicaux.

La présente invention sera mieux comprise à l'aide des exemples qui suivent, qui se lisent en regard des figures en annexe; ces exemples sont donnés uniquement à titre d'illustration de l'objet de l'invention, .
La figure 1 est un spectre infrarouge des dérivés polysaccharidiques HE 800 ayant subi une dépolymérisation radicalaire, avant (HE 800 DR) et après la réaction de N-désacétylation (HE 800 DRN) ;
La figure 2 représente les spectres infrarouges du dérivé HE 800 ayant subi une dépolymérisation radicalaire (dérivé HE 800 DR) et du dérivé HE 800 N-desacétylé et sulfaté (HE 800 DRNS) ;
La figure 3 est un graphe qui montre l'effet du dérivé GY785 dépolymérisé radicalairement puis sulfaté, selon l'invention, sur la prolifération de fibroblastes dans un derme reconstruit.
La figure 4 est un ensemble de 6 photos a, b, c, d, e, f. Cette figure concerne un test d'immunodétection, dans une culture fibroblastique dermique, des filaments α-actine, caractéristiques de la sous population de myofibroblastes.
La figure 5 est un graphe qui reflète un résultat obtenu en zymographie, et montre l'effet du HE 800 DRNS sur la sécrétion de MMP-2 par les fibroblastes en culture : la figure 5 montre que la sécrétion de MMP-2 est inhibée.
La Figure 6 est un ensemble de deux photos, a et b, qui montrent que l'effet d'un dérivé conforme à l'invention sur la sécrétion d'une protéase matricielle, la stromélysine (MMP-3).

### EXEMPLE 1 : COMPOSITION OSIDIQUE DES EPS NATIFS BACTERIENS

### Méthodes :

La teneur en protéine a été déterminée *selon la méthode du BCA* (acide bicinchoninique) décrite par Wiehelman, K. et al. (Anal. Biochem. 1988, 175 : 231-237).

La teneur en oses neutres a été déterminée par la méthode de Tillmans et Philippi (Analyt. Chem., 1929, 28 : 350) modifiée par Rimington (Biochem. J., 1931, 25: 1062-1071).

La teneur en acides uroniques (GlcA) a été établie en utilisant une modification de la méthode m-hydroxydiphényle-H₂SO₄ (Filisetti-Cozzi et Carpitta, Anal. Biochem., 1991, 197: 157-162) et en utilisant l'acide glucuronique comme étalon. L'interférence d'hexoses neutres a été évitée en utilisant du sulfamate de potassium et en procédant à des contrôles comprenant tous les réactifs à l'exception du m-hydroxydiphényle.

Les teneurs en oses neutres et acides ont été déterminées par chromatographie en phase gazeuse. L'analyse des résidus glycosidiques sous forme de dérivés triméthylsilylés a été réalisée selon la méthode de Kamerling et al. (Biochem. J., 1975, 151 : 491-495) et modifiée par Montreuil et al. (Glycoproteins In : Carbohydrate analysis, a practical approach, 1986, Chaplin M.F. and Kennedy J.F. (eds), IRL Press, Oxford, 143-204).

La teneur en hexosamines et N-acétylhexosamines (GalNAc et GlcNAC) est déterminée par la méthode de Belcher et al., (Analyst, 1954, 79 : 201-208) adaptée de celle de Elson and Morgan (Biochem J, 1933, 27 :1824-1828) et en utilisant la N-acétylglucosamine et la glucosamine comme étalons.

Les contenus en sulfates totaux (libres plus liés des EPS natifs) ont été déterminés par analyse élémentaire du soufre (S %), et en appliquant la relation suivante : pourcentage de groupes sulfate (%) = 3,22 x S %. Le taux de sulfates libres est quantifié par chromatographie échangeuse d'ions sur un système Dionex® DX-500 relié à un conductimètre et suivant la méthode décrite par le fabricant Dionex. Le résultat obtenu permet de calculer le taux de sulfates réellement liés au dérivé d'EPS qui est égal au taux de sulfates totaux (obtenu par analyse élémentaire) moins le taux de sulfates libres (obtenu par chromatographie échangeuse d'ions).

### Description :

| **Souche** | **EPS** | **COMPOSITION OSIDIQUE** | | | **Sulfates** |
|---|---|---|---|---|---|
| | | **Neutre %** | **Acide %** | **Osamine %** | **%** |
| HYD 1545^{1,2,3} | 1545^{1,2,3} | 49 | 34 | 0.2 | 11 |
| *Alteromonas sp* | | | | | |
| HYD 721^{1,2,12} | 721^{1,2,12,16} | 55 | 11 | < 0.5 | 12 |
| *Pseudoalleromonnas* sp | | | | | |
| HYD 1644^{2,7,8} | 1644^{2,7,8,16} | 55 | 35 | < 1 | 5 |
| *Alteromonas sp* | | | | | |
| HYD 657^{2,14} | 657^{2,14} | 47 | 26 | 1.6 | 5 |
| *Alteromonas macleodii* sp *supsp fijiensis* biovar deepsane | | | | | |
| ST 716^{4,9,10} | 716^{9,10,16} | 40 | 40 | < 1 | 5 |
| *Alteromonas macleodii sp* subsp. *Fijiensis* | | | | | |
| GY 785⁵ | GY 785^{5,11,15,16} | 55 | 40 | < 0.7 | 10 |
| *Alteromonas infernus sp* | | | | | |
| MS 907¹⁶ | MS 907¹⁶ | 50 | 37 | 0 | 0 |
| *Alteromonas macleodii sp* subsp *fijiensis* biovar medioatlantica | | | | | |
| HE 800^{6,9,13} | HE 800^{6,9,13} | 1 | 32 | 30 | 0 |
| *Vibrio diabolicus sp* | | | | | |

| | | | | | |
|---|---|---|---|---|---|
| **1**) (Aymard et al., 1991 *Food Hydrocoll,* **5**, 167-169); **2**) (Guezennec et al., 1994 *Carbohydr. Polym.,* **24**, 287-294) ; **3**) (Vincent et al., 1994 *Appl. Environ. Microb.,* **60**, 4134-4141) ; **4**) Raguenes et al, 1996 *Appl Env Microbiol,* **62**, 67-73) ; **5**) (Raguenes et al., 1997 *Journal of Systematic Bacteriology,* **47**, 989-995); **6**) (Raguenes et al., 1997 *J. Appl. Microbiol.,* **82**, 422-430); **7**) (Dubreucq et al., 1996 *Carbohydr Res*, **290**, 175-81) ; **8**) (Bozzi et al., 1996 *Int J Biol Macromol,* **18**, 9-17); **9**) (Rougeaux et al., 1996 *Carbohydr. Polym.,* **31**, 237-242); **10**) (Rougeaux et al., 1998 *Carbohydr. Res.,* **312**, 53-59); **11**) (Guezennec et al., 1998 Carbohydr. Polym. 37, 19-24.) ; **12**) (Rougeaux et al., 1999 Carbohydr Res., 315, 273-285); **13**) (Rougeaux et al., 1999 Carbohydr. Res., 322, 40-45); **14**) Cambon-Bonavita et al, 2002 J Applied Microbiol, 93, 310-315) ; **15**) (Guezennec, 2002 J Ind Microbiol Biot, 29, 204-208) ; **16**) (Raguenes et al, 2003 Curr Microbiol, 46, 448-52); **17**) (Roger et al, 2004. Res., 339, 2371-2380) | | | | | |

### EXEMPLE 2 : PREPARATION DE DERIVES SELON L'INVENTION A PARTIR DE L'EPS HE 800 NATIF

(1) HE 800 DR correspond au dérivé polysaccharidique, de faible masse molaire
(2) HE 800 DRS correspond au dérivé polysaccharidique de faible masse molaire et sulfaté,
(3) HE 800 DRNS correspond au dérivé polysaccharidique de faible masse molaire, N-désacétylé et sulfaté.

Les dérivés polysaccharidiques de faible masse molaire et sulfatés conformes à l'invention sont obtenus : (i) pour la série DRS en appliquant une première étape de dépolymérisation radicalaire (DR) et d'une étape de sulfatation (S) et (ii) pour la série DRNS en appliquant une première étape de dépolymérisation radicalaire (DR) suivie d'une étape de N-désacétylation (N) et d'une étape de sulfatation (S).

### 2.1. Dépolymérisation radicalaire d'un EPS natifs

500 mg d'EPS de la bactérie marine d'origine hydrothermale HE 800 sont produits selon le procédé décrit dans EP 975 791 et lyophilisés. L'EPS natif est lentement réhydraté durant une nuit dans 100 ml d'eau et est introduit dans un réacteur verre à double paroi. Le catalyseur métallique est ajouté au milieu réactionnel sous forme d'une solution d'acétate de cuivre à 16 mg/mL. La température du milieu est portée à 60°C. Une agitation magnétique est maintenue durant toute la réaction. Le milieu réactionnel est amené à un pH compris entre 7,5 et 8 avec de la soude concentrée 10N. Le pH du milieu réactionnel est suivi et sa régulation est assurée par l'addition d'une solution de soude.

Le peroxyde d'hydrogène (H₂O₂) est ensuite ajouté dans le réacteur à un débit de 1 ml/min à l'aide d'une pompe péristaltique. Le peroxyde d'hydrogène est préparé extemporanément à partir d'une solution concentrée.

### Réduction :

1 g de borohydrure de sodium pour 1 g de dérivé polysaccharidique est dissous dans un petit volume d'eau puis ajouté directement dans un réacteur. La réaction se déroule à température ambiante et sous agitation sur une durée de 2 à 20 heures. Elle est arrêtée par l'addition d'acide acétique 10 N. Un précipité noirâtre dû au cuivre se forme au cours de la réaction.

### Elimination du catalyseur :

Afin d'éliminer le précipité formé lors de la réaction de réduction, la solution contenant le dérivé polysaccharidique est filtré sur büchner équipé de filtres en microfibres de verre de 3µm. Le cuivre résiduel est ensuite éliminé par passage de la solution contenant le dérivé polysaccharidique sur une résine Chelex™, de capacité 0,4 meq/ml. La solution contenant le dérivé polysaccharidique percole à un débit de 4 à 5 ml/min sur une colonne (25 x 400 mm) de 200 ml de résine préalablement passivée. En sortie, la solution contenant le dérivé polysaccharidique a un pH basique de 10.

### Diafiltration, Concentration par Ultra-Filtration et lyophilisation :

La solution contenant le dérivé polysaccharidique est ultrafiltrée sur un système d'ultrafiltration Pellicon 2 (Millipore) équipé d'une membrane Pall de 1 000 g/mol. La conductivité de la solution (4 à 5 mS) est mesurée tout au long de la diafiltration. Lorsqu'une valeur stable et inférieure à 100 µS est atteinte sur le filtrat, la solution est revenue à un pH neutre. Elle peut alors être concentrée puis lyophilisée (Lyophilisateur CIRP). Une fois lyophilisé, le dérivé polysaccharidique obtenu est caractérisé.

### 2.2. N-désacétylation

*Principe :* Dans le but de substituer à la fois les unités osidiques en groupements N- et O-sulfate, le dérivé polysaccharidique HE 800 DR est N-désacétylé. Le procédé de N-désacétylation du dérivé HE 800 est réalisé sur des quantités importantes de produit.

### Méthode :

259 mg d'EPS HE 800 DR sont solubilisés dans 10 ml d'eau et placés dans un ballon sous agitation magnétique. 263 mg de NaBH₄ sont solubilisés dans 1,25 ml d'eau puis ajoutés à la solution d'EPS HE 800 DR. Lorsque la température du mélange atteint 80°C, 1,25 ml de NaOH 10 N sont ajoutés. La concentration finale de la solution est alors de 1N en soude et de 2 % en NaBH₄ pour un volume total de 12.5 ml.

Après une heure de réaction, la solution est neutralisée avec de l'acide acétique 10N jusqu'à arrêt de l'effervescence. Le volume ajouté est de 1.5 ml et le pH est de 5. La solution est ensuite ultrafiltrée sur membrane de 1 000 g/mol puis lyophilisée. 167 mg d'EPS HE 800 DR N-désacétylé (HE 800 DRN) sont obtenus reflétant un rendement de 65 %.

### 2.3. Sulfatation de l'EPS HE 800 DR ou de HE 800 DRN

### Préparation du polysaccharide sous forme de sel :

50 mg d'EPS sont solubilisés dans 20 ml d'H₂O. Le dérivé polysaccharidique est mis sous forme H+ par élution sur une colonne de résine Dowex. L'élution est réalisée avec de l'eau, l'éluat est collecté tant que le pH reste acide, de préférence inférieur à 5. Le pH est immédiatement ajusté à 6.5 avec la base désirée, (pyridine, triétylamine, tributhylamine, soude). Le dérivé polysaccharidique sous forme de sel est alors lyophilisé.

### Sulfatation du polysaccharide :

Le dérivé polysaccharidique sous forme de sel est dissous dans 100 ml de DMF anhydre sous agitation douce (250 tours/min) pendant 2 heures à température ambiante, puis pendant 2 heures à une température de 45 °C.

Lorsque la dissolution est complète, 2,5 g de complexe de pyridine-SO₃ sont ajoutés au milieu réactionnel, soit 5 fois la masse du polysaccharide. La température du mélange est ensuite maintenue à 45 °C pendant 2 heures sous agitation. La réaction est terminée par addition de 40 ml d'eau et de soude afin d'obtenir un pH à 9. Le mélange réactionnel est alors dialysé dans l'eau avec un boudin de dialyse présentant un seuil de coupure de 3 500 Da.

Après dialyse, la solution contenant l'EPS sulfaté est filtrée sur des filtres de 2,7 µm puis 0,7 µm et ultrafiltrée sur membrane de 1 000 g/mol puis lyophilisée.²

### 2.4. Caractérisation des différents dérivés : (1) HE 800 DR ; (2) HE 800 DRS et (3) HE 800 DRNS.

Les masses molaires (Mc : Masse molaire chromatographique déterminée au sommet du pic ; Mp ou Mw : Masse molaire moyenne en poids et Mn : Masse molaire moyenne en nombre) et la polydispersité (I = Mp/Mn) des différents dérivés d'EPS HE 800 obtenus ont été déterminées par chromatographie d'exclusion stérique haute performance (HPSEC) sur une chaîne Biotech, dans l'acétate d'ammonium aqueux 0,1 M à un débit de 0,1 ml/min en utilisant une colonne Superdex® 200 ou une colonne Superdex™ Peptide (AMERSHAM). La colonne a été calibrée avec des étalons polysaccharidiques suivants : pullulanes : 758 000-5900 g/mol (Polymer Laboratories, Interchim), polysaccharides standards non commerciaux : 4 000 ; 3 000 et 1500 g/mol ; mélézitose : 522 g/mol (FLUKA), sucrose : 342 g/mol ; glucose : 180 g/mol (SIGMA). Les résultats sont analysés en utilisant le logiciel Aramis® (Varian, France).

La teneur en oses neutres a été déterminée par la méthode de Tillmans et Philippi (Analyt. Chem., 1929, 28 : 350-) modifiée par Rimington (Biochem. J., 1931, 25 : 1062-1071).

La teneur en acides uroniques (GlcA) a été établie en utilisant une modification de la méthode m-hydroxydiphényle-H₂SO₄ (Filisetti-Cozzi et Carpitta, Anal. Biochem., 1991, 197 : 157-162) et en utilisant l'acide glucuronique comme étalon. L'interférence d'hexoses neutres a été évitée en utilisant du sulfamate de potassium et en procédant à des contrôles comprenant tous les réactifs à l'exception du m-hydroxydiphényle.

La teneur en hexosamines et N-acétylhexosamines (GalNAc et GlcNAc) est déterminée par la méthode de Belcher et al., (Analyst, 1954, 79 : 201-208) adaptée de celle de Elson and Morgan (Biochem J, 1933, 27 :1824-1828) et en utilisant la N-acétylglucosamine et la glucosamine comme étalon.

Les contenus en sulfates totaux (libres plus liés) ont été déterminés par analyse élémentaire du soufre (S %), et en appliquant la relation suivante : pourcentage de groupes sulfate (%) = 3,22 x S %.

Le taux de sulfates libres est quantifié par chromatographie échangeuse d'ions sur un système Dionex® DX-500 relié à un conductimètre et suivant la méthode décrite par le fabricant Dionex. Le résultat obtenu permet de calculer le taux de sulfates réellement liés au dérivé d'EPS qui est égal au taux de sulfates totaux (obtenu par analyse élémentaire) moins le taux de sulfates libres (obtenu par chromatographie échangeuse d'ions).

La spectroscopie Infrarouge à transformée de Fourier (FT-IR) a été réalisée sur un Vector 22 possédant une résolution de 4 cm⁻¹. Les spectres infrarouges des polysaccharides ont été réalisés en pastilles de KBr (2 mg de polysaccharide sont mélangés à 200 mg de KBr sec), tous les spectres infrarouges ont été enregistrés entre 4000 et 400 cm⁻¹.

### 2.5. Résultats

*Composition d'un dérivé HE 800 DR :*

| **Caractéristiques** | **Dérivé HE 800 DR** |
|---|---|
| Oses neutres (g/100 g)¹ | 0 |
| Oses acides (g/100 g)¹ | 40 |
| Hexosamines (g/100 g)¹ | 40 |
| -SO₃Na total (g/100 g)² | 0 |
| Mc (g/mol)⁴ | 15 000 |
| Mp (g/mol)⁴ | 29 000 |
| Mn (g/mol)⁴ | 13 000 |
| I (Mp/Mn)⁴ | 2,2 |

| | |
|---|---|
| ¹: Dosages colorimétriques. ² : Dosage par analyse élementaire. ³ Dosage par chromatographie échangeuse d'ions. ^{4.} : Déterminé par chromatographie HPSEC en équivalent Pullulanes. | |

*Composition d'un dérivé EPS HE 800 DRS :*

| **Caractéristiques** | **Dérivé HE 800 DRS** |
|---|---|
| Oses neutres (g/100 g)¹ | 0 |
| Oses acides (g/100 g)¹ | 30 |
| Hexosamines (g/100 g)¹ | 30 |
| -SO₃Na total (g/100 g)² | 25 |
| Mc (g/mol)⁴ | 4 800 |
| Mp (g/mol)⁴ | 5 800 |
| Mn (g/mol)⁴ | 4 500 |
| I (Mp/Mn)⁴ | 1,3 |

| | |
|---|---|
| ¹ : Dosages colorimétriques. ² : Dosage par analyse élémentaire. ^{3.} : Dosage par chromatographie échangeuse d'ions. ^{4.} : Déterminé par chromatographie HPSEC en équivalent Pullulanes. | |

*Composition d'un dérivé EPS HE 800 DRNS:*

| **Caractéristiques** | **Dérivé HE 800 DRNS** |
|---|---|
| Oses neutres (g/100 g)¹ | 0 |
| Oses acides (g/100 g)¹ | 20 |
| Hexosamines (g/100 g)¹ | 20 |
| -SO₃Na total (g/100 g)² | 34 |
| Mc (g/mol)⁴ | 22 000 |
| Mp (G/mol)⁴ | 27 000 |
| Mn (g/mol)⁴ | 19 000 |
| I (Mp/Mn)⁴ | 1,4 |

| | |
|---|---|
| ¹ : Dosages colorimétriques. ² : Dosage par analyse élémentaire. ^{3.} : Dosage par chromatographie échangeuse d'ions. ^{4.} : Déterminé par chromatographie HPSEC en équivalent Pullulanes. | |

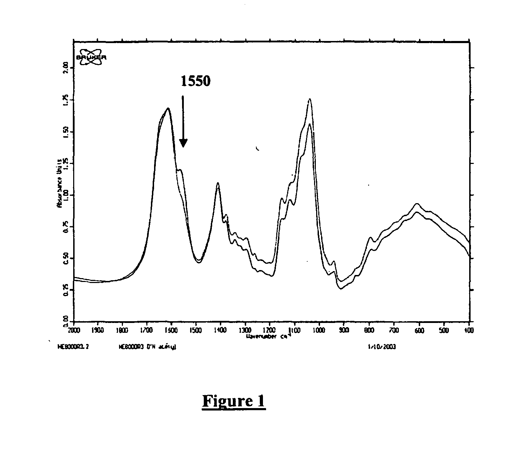

La figure 1 montre les spectres infrarouge des dérivés polysaccharidiques, avant (HE 800 DR) et après la réaction de N-désacétylation (HE 800 DRN). Les spectres FT-IR de la figure 1 ont été enregistrés sur un spectrophotomètre Bruker Vector 22 (résolution de 4 cm⁻¹) 2 mg de dérivé d'EPS HE800 ont été traités avec 200 mg de KBr lors de l'étape de N-désacétylation. L'analyse des spectres infrarouge des dérivés, avant et après l'étape de N-désacétylation, montre à la fréquence de 1 550 cm -1 la perte d'une bande d'absorption caractéristique des groupements N-acétylés (Figure 1).

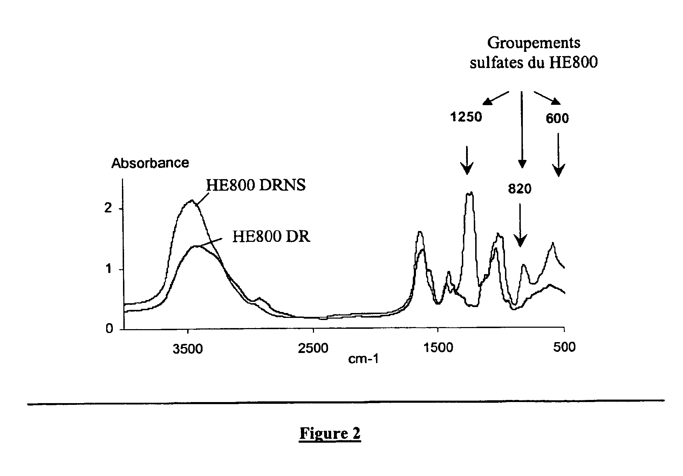

La figure 2 montre les spectres infrarouges (IR) du dérivé polysaccharidique HE 800 non sulfaté (HE800DR) et du dérivé polysaccharidique HE 800 N-désacétylé et sulfaté (HE 800 DRNS). L'apparition des bandes correspondant à la présence d'ester sulfate (1250, 820 et 600 cm⁻¹) est observée pour le dérivé polysaccharidique HE 800 DRNS.

### EXEMPLE 3 : PREPARATION DE DERIVES SELON l'INVENTION A PARTIR DE L'EPS GY 785 NATIF

(1) GY 785 DR correspond au dérivé polysaccharidique de faible masse molaire, obtenu après une étape de dépolymérisation.
(2) GY 785 DRS correspond au dérivé polysaccharidique de faible masse molaire, obtenu après une étape de dépolymérisation suivie d'une étape de sulfatation.
1) Dépolymérisation radicalaire et réduction au borohydrure de sodium
400 mg d'EPS GY 785 sulfaté obtenu ci-dessus à l'étape précédente ont été dissous dans 95 ml d'eau. Après dissolution, on a ajouté 2 ml d'une solution catalytique contenant 36 mg d'acétate de cuivre monohydraté (10⁻³ M). La température du réacteur est alors portée à 60°C et le pH est ajusté à 7,5 par addition de soude 1 M. Une solution à 0,115 % (v/v) de peroxyde d'hydrogène a alors été ajoutée à un débit de 1 ml par minute, et le pH a été régulé autour de 7,5 par addition de soude 1 M. La réaction a été arrêtée au bout de 1 heure.
La réduction est réalisée en fin de dépolymérisation par addition dans le réacteur de borohydrure de sodium (270 mg de Na BH₄ dissous dans 10 ml d'eau). La réduction se déroule sous agitation pendant 2 heures à température ambiante. La réduction est stoppée par addition d'acide acétique 10 N qui permet d'éliminer l'excès de Na BH₄ restant sous forme de dégagement gazeux d'hydrogène. La solution a ensuite été filtrée sur Büchner avec des filtres en microfibres de verre (porosité 3 µm). La solution filtrée a été éluée sur une colonne CHELEX® 20 (BIORAD) afin d'éliminer le cuivre résiduel. La solution décontaminée est ensuite ultrafiltrée sur cassette (seuil de coupure 1 000 Da) puis lyophilisée.
2) Sulfatation chimique de l'EPS GY 785
500 mg de lyophilisat d'EPS GY 785 produit par la bactérie marine d'origine hydrothermale *Alteromonas infernus* selon le procédé décrit à l'exemple 1 du brevet FR 2 755 142, ont été dissous dans 100 ml de DMF anhydre sous agitation douce (250 tours/min) pendant 2 heures à température ambiante, puis pendant 2 heures à une température de 45 °C.
Lorsque la dissolution est complète, 2,5 g de complexe de pyridine-SO₃ vendu sous la référence 84737 par la société Fluka (soit 5 fois la masse du polysaccharide GY 785) ont été ajoutés au milieu réactionnel. La température du mélange a ensuite portée et maintenue à 45 °C pendant 2 heures sous agitation. Le mélange réactionnel a été transféré dans un bécher. La réaction a alors été stoppée par addition de 40 ml d'eau, puis le pH a été amené à 9 avec de la soude 3 M. Le mélange réactionnel a alors été dialysé sur un boudin de dialyse présentant un seuil de coupure compris entre 12000 et 16 000 Da contre de l'eau du robinet (1 nuit avec de l'eau courante) puis 3 fois pendant 24 heures contre de l'eau Milli-Q.
Après dialyse, la solution contenant l'EPS GY 785 sulfaté a été congelée et lyophilisée.
Les caractéristiques des dérivés EPS DR et EPS DRS ont été déterminées selon les méthodes décrites ci-dessus à l'exemple 1 et sont résumées dans le Tableau ci-après :

| **Caractéristiques** | **EPS DR** | **EPS DRS** |
|---|---|---|
| Oses totaux (g/100 g)¹ | 51 | nd |
| Oses acides (g/100 g)¹ | 38 | nd |
| -SO₃Na total (g/100 g) ² | 10 | 42 |
| Mc (g/mol) | 7800 | 13000 |
| Mp (g/mol) | 17300 | 23600 |
| Mn (g/mol) | 6000 | 8800 |
| I(Mp/Mn) | 2,8 | 2,7 |
| Activité anticoagulante³ | inactif | 7 |

| | | |
|---|---|---|
| ¹ : Dosages colorimétriques. ² : Dosage par analyse élémentaire.³ : Quantité de polysaccharide en µg/ml de plasma humain nécessaire pour doubler le temps de coagulation contrôle, TCA (temps contrôle = 40 secondes) ; nd : non déterminé | | |

*Composition d'un autre dérivé GY 785 DR*

| **Caractéristiques** | **Dérivé GY 785 DR** |
|---|---|
| Oses neutres (g/100 g)¹ | 40 |
| Oses acides (g/100 g)¹ | 15 |
| Hexosamines (g/100 g)¹ | 0 |
| -SO₃Na total (g/100 g)² | 10 |
| Mc (g/mol)⁴ | 16 000 |
| Mp (G/mol)⁴ | 40 000 |
| Mn (g/mol)⁴ | 13 000 |
| 1 (Mp/Mn)⁴ | 3 |

| | |
|---|---|
| ¹ Dosages colorimétriques. ² : Dosage par analyse élémentaire. ^{3.} : Dosage par chromatographie échangeuse d'ions. ^{4.} : Déterminé par chromatographie HPSEC en équivalent Pullulanes. | |

*Composition d'un autre dérivé GY 785 DRS*

| **Caractéristiques** | **Dérivé GY 785 DRS** |
|---|---|
| Oses neutres (g/100 g) ¹ | 20 |
| Oses acides (g/100 g)¹ | 10 |
| Hexosamines (g/100 g)¹ | 0 |
| -SO₃Na total (g/100 g)² | 45 |
| Mc (g/mol)⁴ | 23 000 |
| Mp (G/mol)⁴ | 29 000 |
| Mn (g/mol)⁴ | 21 000 |
| I (Mp/Mn)⁴ | 1,4 |

| | |
|---|---|
| ¹ : Dosages colorimétriques. ² : Dosage par analyse élémentaire. ^{3.} : Dosage par chromatographie échangeuse d'ions. ^{4.} : Déterminé par chromatographie HPSEC en équivalent Pullulanes. | |

### EXEMPLE 4 : PREPARATION DE DERIVES SELON L'INVENTION A PARTIR DE L'EPS HYD 721 NATIF

(1) HYD 721 DR correspond au dérivé polysaccharidique de faible masse molaire, obtenu après une étape de dépolymérisation,
(2) HYD 721 DRS correspond au dérivé polysaccharidique de faible masse molaire et sulfaté par sulfatation chimique selon l'invention.

Le dérivé polysaccharidique sulfaté HYD 721 est obtenu en mettant en oeuvre le procédé tel que décrit dans l'exemple 2. Toutefois dans la mesure où l'EPS HYD 721 natif ne renferme pas d'hexosamines N-acétylées, le procédé de préparation du dérivé sulfaté ne comprend pas d'étape de N-désacétylation.

*Composition d'un dérivé HYD 721 DR*

| **Caractéristiques** | **Dérivé HYD 721 DR** |
|---|---|
| Oses neutres (g/100 g)¹ | 68 |
| Oses acides (g/100 g)¹ | 17 |
| Hexosamines (g/100 g)¹ | 0 |
| -SO₃Na total (g/100 g)² | 11 |
| Mc (g/mol)⁴ | 10 000 |
| Mp (G/mol)⁴ | 12 000 |
| Mn (g/mol)⁴ | 7 000 |
| 1 (Mp/Mn)⁴ | 1,7 |

| | |
|---|---|
| ¹ : Dosages colorimétriques. ² : Dosage par analyse élémentaire. ^{3.} : Dosage par chromatographie échangeuse d'ions. ^{4.} : Déterminé par chromatographie HPSEC en équivalent Pullulanes. | |

*Composition d'un dérivé HYD 721 DRS*

| **Caractéristiques** | **Dérivé HYD 721 DRS** |
|---|---|
| Oses neutres (g/100 g)¹ | 35 |
| Oses acides (g/100 g)¹ | 5 |
| Hexosamines (g/100 g)¹ | 0 |
| -SO₃Na total (g/100 g)² | 43 |
| Mc (g/mol)⁴ | 17 500 |
| Mp (G/mol)⁴ | 20 000 |
| Mn (g/mol)⁴ | 10 000 |
| I (Mp/Mn)⁴ | 2 |

| | |
|---|---|
| ¹ : Dosages colorimétriques. ² : Dosage par analyse élémentaire. ^{3.} : Dosage par chromatographie échangeuse d'ions. ^{4.} : Déterminé par chromatographie HPSEC en équivalent Pullulanes. | |

### EXEMPLE 5: EFFET DES DERIVES SELON L'INVENTION SUR LA PROLIFERATION DES FIBROBLASTES DERMIQUES ET GINGIVAUX

Les dérivés polysaccharidiques sulfatés utilisés dans les essais de prolifération ont été préparés et caractérisés selon les protocoles des exemples 2 et 3.

### 5.1. Cellules en culture bidimensionnelle

Les cellules sont ensemencées dans des boites de culture à raison de 10 000 cellules par puits dans un milieu de culture Dubelco MEM Glutamax I contenant 100 U/ml de pénicilline, 100 µg/ml de streptomycine, 2 µg/ml de fungizone et supplémenté avec 10% de sérum de veau foetal (SVF).

Après adhésion et étalement des cellules pendant 12 heures, le milieu de culture est remplacé par un milieu de culture supplémenté ou non par des différentes concentrations d'un dérivé polysaccharidique sulfaté de faible masse molaire : (i) l'EPS GY785 (GY785 DRS) ou (ii) l'EPS HE 800 (HE 800 DRNS)) HE800. Les cellules sont ensuite comptées après 2, 4, 7 et 10 jours de culture. Les témoins correspondent à des cultures de cellules en absence de dérivés conformes à l'invention (*).

Effet du dérivé GY 785 DRS sur la prolifération de fibroblastes dermiques en culture bidimensionnelle :

| | Jour 2 | Jour 4 | Jour 7 | Jour 10 |
|---|---|---|---|---|
| Témoin* | 100 | 100 | 100 | 100 |
| 0,1 µg/ml GY 785 DRS | 94,5 | 100,0 | 145,3 | 151,4 |
| 1 µg/ml GY 785 DRS | 108,8 | 113,7 | 173,0 | 160,1 |
| 10 µg/ml GY 785 DRS | 87,6 | 157,3 | 156,9 | 151,2 |
| 100 µg/ml GY 785 DRS | 77,4 | 121,2 | 115,6 | 123,4 |

Effet du GY 785 DRS sur la prolifération de fibroblastes gingivaux en culture bidimensionnelle :

| | Jour 2 | Jour 4 | Jour 7 | Jour 10 |
|---|---|---|---|---|
| Témoin* | 100 | 100 | 100 | 100 |
| 0,1µg/ml GY 785 DRS | 112,0 | 121,8 | 135,0 | 139,1 |
| 1 µg/ml GY 785 DRS | 99,2 | 101,5 | 122,6 | 133,0 |
| 10µg/ml GY 785 DRS | 90,2 | 124,3 | 160,4 | 183,3 |
| 100µg/ml GY 785 DRS | 77,7 | 95,4 | 118,4 | 161,2 |

Effet du HE 800 DRNS sur la prolifération de fibroblastes dermiques en culture bidimensionnelle :

| | Jour 2 | Jour 4 | Jour 7 | Jour 10 |
|---|---|---|---|---|
| Témoin* | 100 | 100 | 100 | 100 |
| 10µg/ml HE 800 DRNS | 109,00 | 159,96 | 157,70 | 146,56 |
| 100µg/ml HE 800 DRNS | 95,49 | 191,12 | 163,60 | 167,60 |

Les dérivés polysaccharidiques sulfatés conformes à l'invention sont capables de stimuler la prolifération des fibroblastes dermiques et gingivaux en culture bidimensionnelle.

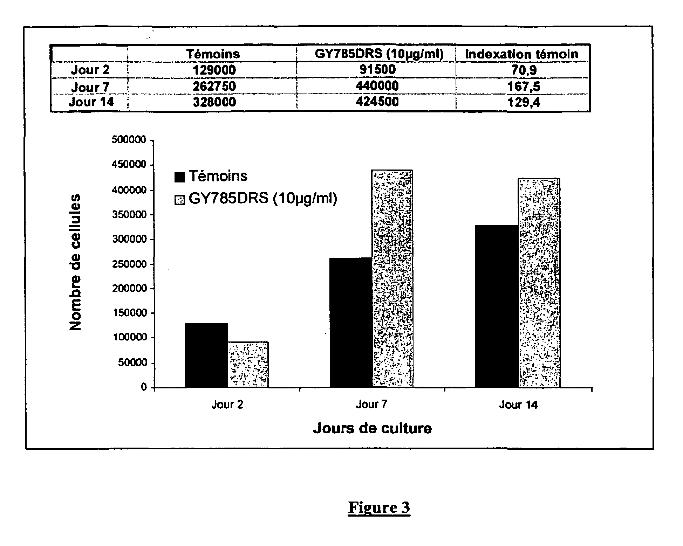

### 5.2. Tissu reconstruit ou lattis (Figure 3)

Les tissus conjonctifs reconstruits ou lattis sont constitués par des fibres de collagène de type I acido-soluble qui après neutralisation polymérisent et forment un gel renfermant des fibroblastes. La préparation d'un lattis est réalisée à froid afin de mieux contrôler la polymérisation des fibres de collagènes.

Ce lattis sous l'influence des cellules va subir de nombreux remaniements appréciables notamment par sa rétractation qui est observée pendant les 15 premiers jours de culture. Ce type de modèle permet d'étudier le comportement des cellules au sein d'un environnement extracellulaire plus proche de l'environnement physiologique que la simple culture sur boite.

La figure 3 révèle que le dérivé polysaccharidique de faible masse molaire et sulfaté GY 785 DRS conforme à l'invention est capable à la concentration de 10 µg/ml de stimuler la prolifération des fibroblastes dans des tissus conjonctifs reconstruits.

### EXEMPLE 6: MISE EN EVIDENCE DE L'EFFET DES DERIVES SELON L'INVENTION SUR LA SELECTION DE LA SOUS POPULATION FIBROBLASTIQUE

Comme pour les essais de prolifération en culture bidimensionnelles, les cellules sont ensemencées dans des boites de culture à raison de 10 000 cellules par puits dans un milieu de culture Dubelco MEM Glutamax 1 contenant 100 U/ml de pénicilline, 100 µg/ml de streptomycine, 2 µg/ml de fungizone et supplémenté avec 10% de sérum de veau foetal (SVF).

Après adhésion et étalement des cellules pendant 12 heures, le milieu de culture est remplacé par un milieu de culture supplémenté ou non par des différentes concentrations d'un dérivé sulfaté obtenu à partir de l'EPS GY785 (GY785 DRS). Les cellules sont ensuite fixées à l'alcool après 2, 4, 7 et 10 jours de culture, l'immunodétection sur ces cultures cellulaires est effectuée comme suit :
Les cellules fixées sont re-perméabilisées dans de l'éthanol 70 (20min), puis réhydratées dans du PBS (10 min). Les peroxydase endogène sont bloquées avec une solution méthanol (30%), H₂O₂ (0,3%). Cette opération est suivie d'un rinçage au PBS (2min) puis d'un blocage des sites antigéniques non spécifiques par une solution PBS/lait écrémé 1% (1h). Les cultures sont alors incubées avec un anticorps primaire (IgG de souris) dirigé contre l'α-actine humaine (1/30; 50min) puis rincées au PBS (3 × 10 min). Les cellules sont alors incubées, au noir pendant 60 min avec un anticorps anti IgG de souris biotinylé (1/200), rinçées au PBS (3 × 10 min) puis incubées avec de la streptavidine couplée à la peroxydase (1/200).
Après rinçage (PBS 3x 10 min), la révélation de l'activité peroxydasique avec la 3-3' diaminobenzidine s'effectue dans un tampon Tris/HCl (100mM, pH 7,2-7,4) contenant 0,1% de H₂O₂ (15 min, à l'obscurité). L'activité peroxydasique fait apparaître un matériel fibrillaire brun qui correspond aux microfilaments d'α actine dans le cytoplasme des cellules positives. Les produits utilisés proviennent de la firme DAKO sous l'appellation DAKOImmuno-détection.

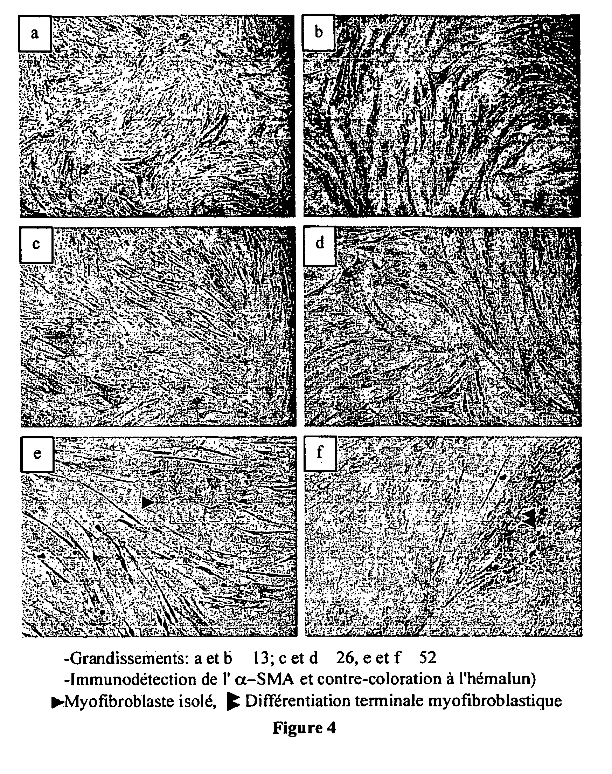

La Figure 4 met en évidence que le dérivé polysaccharidique de faible masse molaire et sulfaté GY 785 DRS conforme à l'invention est capable de stimuler la prolifération des fibroblastes au détriment des myofibroblastes. Les photos a, c et e correspondent à des cultures supplémentées avec 10 µg/ml de dérivé sulfaté dans lesquelles la population dominante est formée de fibroblastes au détriment des myofibroblastes. Les photos b, d et f correspondent à des cultures témoins sans dérivé sulfaté dans lesquelles de nombreux myofibroblastes sont visibles.
Les cultures témoins non traitées par les dérivés conformes à l'invention comprennent un grand nombre de myofibroblastes (α-actine positives), tandis que les fibroblastes n'exprimant pas l'α-actine forment le type cellulaire dominant des cultures traitées.

### EXEMPLE 7 : EFFET DES DERIVES SELON L'INVENTION SUR LA SECRETION DE PROTEASES MATRICIELLES

### 7.1. Protocole

Les cellules sont ensemencées dans des boites de culture à raison de 40000 cellules par puits et menées à confluence dans du milieu de culture Dubelco MEM Glutamax 1 contenant 100 U/ml de pénicilline, 100 µg/ml de streptomycine, 2 µg/ml de fungizone et supplémenté en sérum de veau foetal (SVF).

A confluence, le milieu de culture est remplacé par du milieu ne contenant pas de SVF et supplémenté ou non en IL-1β à une concentration finale de 100 U /ml, en présence ou en absence de dérivés sulfatés.

Les milieux de culture sont prélevés après 48 heures, afin d'étudier la sécrétion par les fibroblastes des MMP en zymographie ou par western-blot. Pour chaque condition expérimentale menées en quadruplicate, deux puits sont fixés à l'éthanol et les deux autres sont trypsinisés afin de détacher les cellules pour les dénombrer.

La sécrétion de métalloprotéases est détectée et quantifiée par zymographie. Il s'agit d'une méthode particulièrement sensible basée sur l'électrophorèse SDS-PAGE effectuée dans des conditions réductrices. Le substrat de la MMP-2, la gélatine copolymérise avec l'acrylamide. Après migration, le SDS est éliminé par des lavages dans une solution de Triton X-100 permettant la restauration de l'activité de la MMP-2. Le gel est ensuite incubé à 37 °C dans un tampon d'incubation (Tris/HCl 0.1 M pH 7,4, CaCl₂ 30 mM, NaN₃ 0,001%, Brij 0,0015% ZnCl₂ 0,1 µM).

Après coloration (bleu de coomassie (0,5%) acide acétique (10%), Isopropanol (30%), puis décoloration (acide acétique (10%), Methanol (40%) eau distillée (50%), les bandes illustrant l'activité de la métalloprotéase MMP-2 apparaissent décolorées. La teinte de gris et la surface de ces bandes sont quantifiées à l'analyseur d'image, le rapport [(teinte de gris × surface)/nombre de cellules] permet une quantification des activité gélatinolytique et la comparaison entre les cultures témoin et les cultures contenant l'exopolysaccharide.

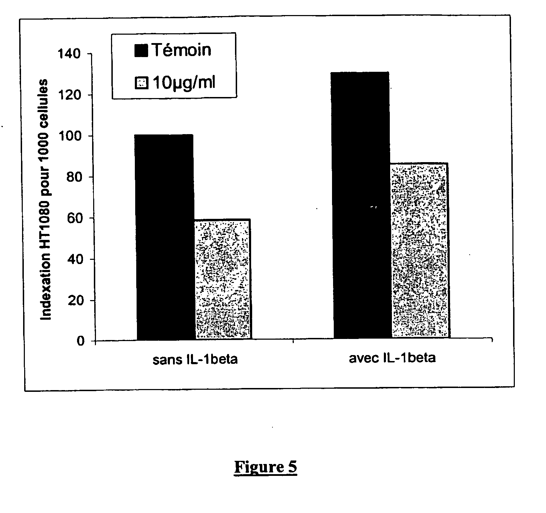

### 7.2. Effet du HE 800 DRNS sur la sécrétion de MMP-2 par les fibroblastes en culture, résultat obtenu en Zymographie (figure 5)

Le graphe de la figure 5 montre que la sécrétion de MMP-2 est inhibée lorsque les fibroblastes sont cultivés en présence de dérivés polysaccharidiques de faible masse molaire et sulfatés, en présence ou absence d'IL1β.

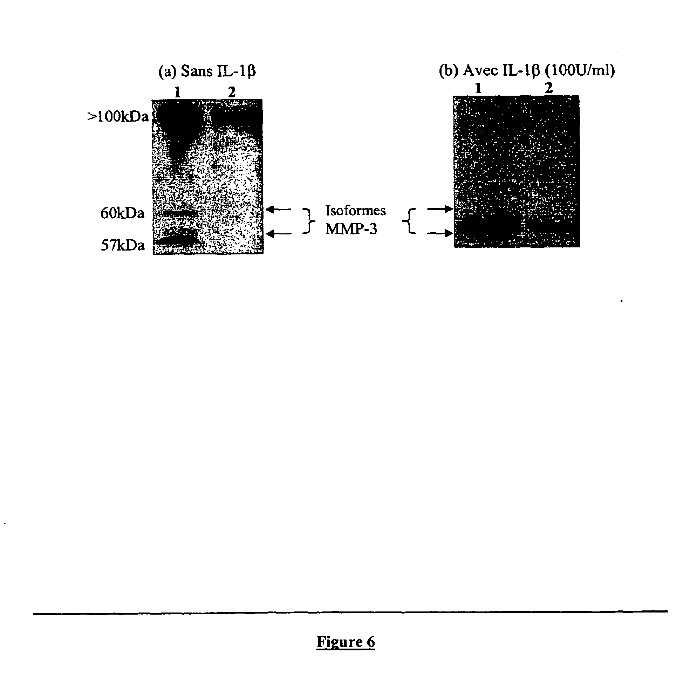

### 7.3. Effet du HE800 DRNS sur la sécrétion de la stromélysine 1 (MMP-3) par des fibroblastes en culture, résultat obtenu par western blott (figure 6)

La Figure 6 montre que l'addition de HE800 DRNS diminue fortement la sécrétion de MMP-3 par les fibroblastes, que cette sécrétion soit basale (photo a) ou induite par une cytokine inflammatoire l'IL-1β (photo b). La photo (a) montre des fibroblastes confluents incubés pendant 48h dans un milieu sans sérum (piste 1), et dans un milieu sans sérum contenant 10 µg/ml de dérivé d'EPS (piste 2) ; la photo (b) fibroblastes confluents incubés pendant 48h dans un milieu sans sérum contenant 100 U/ml d'IL-1β (piste 1), dans un milieu sans sérum contenant 10 µg/ml de dérivé d'EPS et 100 U/ml d'IL-1β (piste 2)
1 : témoin = culture non traitée
2 : culture traitée par 100 U/ml de HE800 DRNS

En conclusion, les dérivés polysaccharidiques sulfatés conforme à l'invention sont capables d'inhiber la sécrétion de protéases matricielles, la gélatinase A (MMP-2) et la stromélysine 1 (MMP-3) des fibroblastes dans des cultures bidimensionnelles.

### EXEMPLE 8: EFFET DES DERIVES SELON L'INVENTION SUR LE COMPLEMENT

### 8.1. Principe

Le complément peut être activé par 3 voies d'activation différentes : la voie classique, la voie alterne et la voie lectine liant le mannose (MBL) aboutissant par des mécanismes différents, à la formation d'enzymes ayant des substrats identiques : les C3/C5 convertases capables d'activer C3 et C5. Les réactions d'activation se déroulent en cascade : un composant acquiert une activité enzymatique qui induit l'activation du composant suivant et ainsi de suite.

Dans la mesure où l'activation finale du complément est commune aux trois voies, seule la voie classique est étudiée dans cet exemple. L'activation de la voie classique intervient lorsque le complexe C1 interagit avec des complexes antigènes-anticorps ou des agrégats immuns contenant des IgG ou des IgM. Le système utilisé pour étudier la voie classique est basé sur l'activation du complément par un complexe immun consistant en des globules rouges de mouton recouverts d'anticorps de lapin. Les anticorps de lapin ayant reconnus comme étrangers, les globules rouges de mouton en présence de sérum humain, déclenchent principalement l'activation de la voie classique. Cette activation entraîne la formation de la C3 convertase, qui déclenche alors l'activation de la voie alterne. L'activation de ces deux voies aboutit à la formation d'un complexe d'attaque membranaire à la surface des globules rouges. Ce complexe induit l'éclatement des globules rouges et la libération d'hémoglobine. La mesure de l'activation du système du complément se fait en dosant la quantité d'hémoglobine libérée, par mesure de l'absorption spectrophotométrique à 414nm. L'activateur (globules rouges de mouton) est également utilisé comme un révélateur de l'activation grâce à l'hémoglobine libérée lors de la lyse cellulaire. La dilution du sérum humain est ajustée pour une quantité donnée de globules, de telle sorte que 50% des cellules soient lysées.

Les globules rouges recouverts d'anticorps sont incubés en absence (témoin) et en présence de différents dérivés polysaccharidiques sulfatés. La quantité d'hémoglobine libérée diminue (diminution de l'absorption à 414nm), traduisant une diminution du nombre de globules rouges lysés et un effet inhibiteur des dérivés polysaccharidiques sulfatés sur l'activation de la voie classique du complément.

350µl de sérum humain normal (NHS) (dilué au 1/100^{e} dans du tampon VBS2+) sont incubés avec 450µl de VBS2+, 200µl d'anticorps de lapin (10⁸ cellules/ml), en présence ou absence de dérivés polysaccharidiques sulfatés.Après un temps de réaction approximatif de 45min à 37°C, une solution de NaCl froid (0.15M) est ajouté et les cellules sont centrifugées à 2400 rpm pendant 10min. L'absorption des surnageants est mesurée à 414nm.

### 8.2. Résultat

Pourcentages d'inhibition du complément (voie classique) en présence de différentes quantités de dérivés d'EPS GY 785 DRS et HE 800 DRNS :

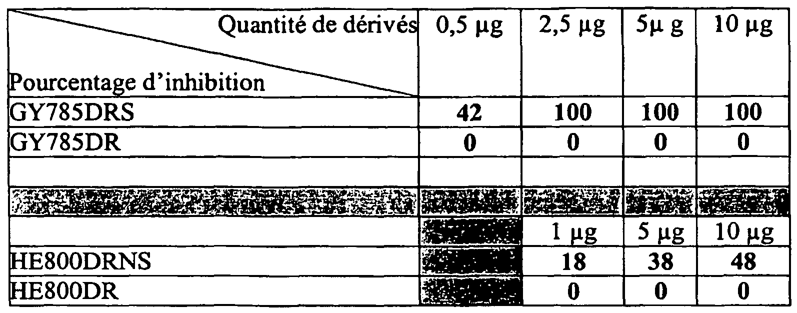

Les dérivés polysaccharidiques de faible masse molaire et sulfatés conformes à l'invention sont capables d'inhiber la voie classique du complément.

### EXEMPLE 9: EFFET DES DERIVES SELON L'INVENTION SUR LA PROLIFERATION DES CELLULES MEDULLAIRES A VOCATION MESENCHYMATEUSE

### 9.1. Protocole

Les cellules stromales sont obtenues à partir de broyat de moelle osseuse, ce broyat est centrifugé pour récupérer un culot cellulaire. Les cellules sont resuspendues dans un milieu de culture composé pour 500ml de milieu contenant 10 % de sérum de cheval, 10 % de sérum de veau foetal, de L-glutamine 200nM (4ml), de MEM vitamine 100X (Gibco brl) (4ml), de Na₂HCO₃ 7,5%, (4ml), d'acides aminés essentiels avec L-glutamine 50X (Gibco Brl) (4ml), de pyruvate de sodium 100X (4ml), d'acides aminés non essentiels 100X (Gibco brl) (1,6ml), le tout étant dilué dans un milieu Mc COY'S 1X (Gibco Brl).

Après 24 heures de culture, seules les cellules ayant adhéré au fond de la boite sont conservées.

Les essais de prolifération sont réalisés dans les mêmes conditions que celles décrites pour les essais de prolifération des cultures de fibroblastes humains gingivaux et dermiques. Les comptages cellulaires sont effectués après 2, 4, 7 et 10 jours de culture. Les immunodétections dirigés contre certains marqueurs phénotypiques (voir protocole α-actine) ont montré que ces cellules n'expriment pas un marqueur caractéristique des macrophages, le CD68 ; une minorité de ces cellules exprime un marqueur leucocytaire le CD45 mais en revanche, elles expriment une protéine du cytosquelette caractéristique des cellules mésenchymateuses, la vimentine. Par ailleurs, une partie de ces cellules sont capables d'exprimer sans stimulation les collagènes de type 1 et III caractéristiques des cellules mésenchymateuse en culture.

### 9.2. Résultat

Effet du GY 785 DRS sur la prolifération de cellules médullaires à vocation mésenchymateuse:

| | Jour 2 | Jour 4 | Jour 7 | Jour 10 |
|---|---|---|---|---|
| Témoin (culture sans dérivés) | 100 | 100 | 100 | 100 |
| 0,1 µg/ml GY 785 DRS | 93,6 | 129,4 | 127,8 | 130,4 |
| 1 µg/ml GY 785 DRS | 95,5 | 170,3 | 170,0 | 164,8 |
| 10µg/ml GY 785 DRS | 79,2 | 134,5 | 155,9 | 179,8 |
| 100µg/ml GY 785 DRS | 86,2 | 129,3 | 139,4 | 167,9 |

La prolifération des cellules médullaire à vocation mésenchymateuse est fortement stimulée en présence de l'EPS GY 785 DRS

## Revendications

1. Dérivés polysaccharidiques de faible masse molaire et sulfatés d'exopolysaccharides natifs (EPS) marins excrétés par des bactéries marines mésophiles issues du milieu hydrothermal profond appartenant au genre *Alteromonas* ou *Vibrio* pour utilisation comme agents cicatrisants des tissus conjonctifs, notamment des tissus conjonctifs dermiques et gingivaux, lesdits dérivés étant susceptibles d'être obtenus par le procédé comprenant les étapes suivantes :
- une étape de dépolymérisation radicalaire desdits EPS natifs pour l'obtention de dérivés dépolymérisés de faible masse molaire, inférieure et ou égale à 100 000 g/mol,
- une étape ultérieure de sulfatation des dérivés dépolymérisés, éventuellement lyophilisés, comprenant l'addition d'au moins un agent de sulfatation en une quantité suffisante pour obtenir des dérivés polysaccharidiques sulfatés présentant un taux de substitution en groupements sulfates compris entre 10 et 45% en poids par rapport au poids total du dérivé polysaccharidique sulfaté, ladite étape de sulfatation étant éventuellement suivie d'une étape de dialyse. A

2. Dérivés polysaccharidiques de faible masse molaire et sulfatés pour utilisation selon la revendication 1, **caractérisés par le fait que** le procédé comprend en outre une étape de réduction succédant à l'étape de dépolymérisation radicalaire.

3. Dérivés polysaccharidiques de faible masse molaire et sulfatés pour utilisation selon l'une quelconque des revendications précédentes, **caractérisés par le fait que** le procédé comprend en outre une étape de N-désacétylation des dérivés polysaccharidiques obtenue à l'issue de l'étape de dépolymérisation et/ou à l'issue de l'étape de réduction.

4. Dérivés polysaccharidiques de faible masse molaire et sulfatés pour utilisation selon l'une quelconque des revendications précédentes, **caractérisés par le fait que** le procédé comprend en outre une étape de lyophilisation des dérivés polysaccharidiques, après la dépolymérisation, et /ou après la réduction et/ou après la N-désacétylation et/ou avant ou après la sulfatation.

5. Dérivés polysaccharidiques de faible masse molaire et sulfatés pour utilisation selon la revendication 1, **caractérisés par le fait que** la première étape de dépolymérisation radicalaire est réalisée par addition d'une solution d'un agent oxydant, de préférence choisi parmi les ions Cu⁺⁺, Fe⁺⁺, Cr⁺⁺⁺ et l'anion Cr₂O₇²⁻.

6. Dérivés polysaccharidiques de faible masse molaire et sulfatés pour utilisation selon la revendication 5, **caractérisés par le fait que** ledit agent oxydant est une solution de peroxyde d'hydrogène, de préférence de concentration comprise entre 0,1% et 0,5% en poids, qui est ajoutée à un débit compris entre Vl/1000 et V1/10 ml/minute, de préférence de V1/50 à V1/500 ml/minutes, V1 étant le volume du milieu réactionnel contenant un exopolysaccharide (EPS) marin excrété par des bactéries marines mésophiles issues du milieu hydrothermal profond dans lequel est additionnée la solution de peroxyde d'hydrogène

7. Dérivés polysaccharidiques de faible masse molaire et sulfatés pour utilisation selon l'une quelconque des revendications précédentes, **caractérisés par le fait que** le ou les agents de sulfatation utilisés pour l'étape de sulfatation sont choisis parmi les complexes de pyridine sulfate, de triéthylamine sulfate, diméthylformamide sulfate, ou de triméthylamine sulfate.

8. Dérivés polysaccharidiques de faible masse molaire et sulfatés pour utilisation selon l'une quelconque des revendications précédentes, **caractérisés par le fait que**, pour la réalisation de l'étape de sulfatation, le ou les agents de sulfatation chimique sont ajoutés en une quantité pondérale représentant de 4 à 6 fois la masse des dérivés polysaccharidiques en solution et sont additionnés aux EPS dépolymérisés qui sont sous forme sèche ou sous la forme de solution dans un solvant de préférence anhydre.

9. Dérivés polysaccharidiques de faible masse molaire et sulfatés pour utilisation selon l'une quelconque des revendications précédentes, **caractérisés par le fait qu'**ils présentent un indice de polydispersité inférieur à 5, de préférence compris entre 1,5 et 4 et un taux de substitution en groupements sulfates compris entre 10 et 45% en poids, et de préférence comprise entre 20 et 45% en poids inclusivement.

10. Dérivés polysaccharidiques de faible masse molaire et sulfatés pour utilisation selon la revendication 9, **caractérisés par le fait qu'**ils présentent une masse molaire inférieure ou égale à 25 000 g/mol, un indice de polydispersité inférieur à 2, et un taux de substitution en groupements sulfates compris entre 10 et 45% en poids, et de préférence compris entre 20 et 40% en poids inclusivement.

11. Dérivés polysaccharidiques de faible masse molaire et sulfatés pour utilisation selon l'une quelconque des revendications 1 à 10, **caractérisés par le fait que** les bactéries du genre *Alteromonas* sont de la souche GY785.

12. Dérivés polysaccharidiques de faible masse molaire et sulfatés pour utilisation selon l'une quelconque des revendications 1 à 10, **caractérisés par le fait que** les bactéries du genre *Alteromonas* sont sélectionnées parmi les souches HYD 657, HYD 708, HYD 721, HYD 1545, HYD 1644, ST 716 et MS 907.

13. Dérivés polysaccharidiques sulfatés pour utilisation selon l'une quelconque des revendications 1 à 10, **caractérisés par le fait que** les bactéries du genre *vibrio* sont des bactéries de la souche HE 800.

14. Dérivés polysaccharidiques de faible masse molaire et sulfatés pour utilisation selon l'une quelconque des revendications 1 à 12, **caractérisés par le fait que** lesdits exopolysaccharides (EPS) natifs excrétés par des bactéries du genre *Alteromonas* ont une composition osidique comprenant :
- de 20% à 70%, préférentiellement de 30% à 60%, et plus préférentiellement de 38% à 57% en poids d'oses neutres,
- de 5% à 60%, préférentiellement de 6% à 50%, et plus préférentiellement de 8% à 42% en poids d'oses d'acides, et
- de 0 à 1% en poids d'osamines.

15. Dérivés polysaccharidiques de faible masse molaire et sulfatés pour utilisation selon l'une quelconque des revendications 1 à 10 et 13, **caractérisés par le fait que** lesdits exopolysaccharides (EPS) marins excrétés par des bactéries du genre *Vibrio* ont une composition osidique comprenant :
- de 0 à 5%, préférentiellement de 0% à 1 %, en poids d'oses neutres,
- de 20% à 50%, préférentiellement de 25% à 40%, et plus préférentiellement de 30% à 32% en poids d'oses acides,
- de 20% à 50%, préférentiellement de 25% à 40%, et plus préférentiellement de 30% à 35% en poids d'osamines, et
- de 0 à 15%, préférentiellement de 4% à 8%, et plus préférentiellement de 5% à 6%, en poids de groupements N-acétylés.

16. Dérivés polysaccharidiques de faible masse molaire et sulfatés pour l'utilisation selon l'une des revendications précédentes, **caractérisés par le fait que** lesdits EPS natifs ont une teneur de 0 à 15%, préférentiellement de 0 à 5%, et plus préférentiellement de 0 à 1% en poids de protéines.

17. Dérivés polysaccharidiques pour l'utilisation selon l'une quelconque des revendications précédentes, **caractérisés par le fait que** lesdits dérivés polysaccharidiques sont capables de stimuler la prolifération des fibroblastes dans des cultures bidimensionnelles ou au sein de tissus conjonctifs reconstruits, notamment de tissus conjonctifs dermiques et gingivaux.

18. Dérivés polysaccharidiques pour l'utilisation selon l'une quelconque des revendications précédentes, **caractérisés par le fait que** lesdits dérivés polysaccharidiques permettent de favoriser la reconstruction et le remodelage des tissus conjonctifs, notamment des tissus conjonctifs dermiques et gingivaux.

19. Dérivés polysaccharidiques pour l'utilisation selon l'une quelconque des revendications précédentes, **caractérisés par le fait que** lesdits dérivés polysaccharidiques sont capables d'inhiber la sécrétion de cytokines pro-inflammatoires par les fibroblastes des tissus conjonctifs notamment des tissus conjonctifs dermiques et gingivaux, et en particulier d'inhiber la sécrétion de l'interleukine 1β et/ou du TNF-α.

20. Dérivés polysaccharidiques pour l'utilisation selon l'une quelconque des revendications précédentes, **caractérisés par le fait que** lesdits dérivés polysaccharidiques sont capables d'inhiber la sécrétion de métalloprotéases matricielles par les fibroblastes des tissus conjonctifs notamment des tissus conjonctifs dermiques et gingivaux et en particulier d'inhiber la sécrétion gélatinase A (MMP-2) et de la stromélysine 1 (MMP-3).

21. Dérivés polysaccharidiques pour l'utilisation selon l'une quelconque des revendications précédentes, **caractérisés par le fait que** lesdits dérivés polysaccharidiques sont capables d'inhiber la voie classique du complément.

22. Dérivés polysaccharidiques pour l'utilisation selon l'une quelconque des revendications précédentes, **caractérisés par le fait que** lesdits dérivés polysaccharidiques sont utilisés pour traiter des pathologies inflammatoires, comme des pathologies infectieuses, néoplasiques ou auto-immunes affectant des tissus conjonctifs, notamment des tissus conjonctifs dermiques et gingivaux.

23. Dérivés polysaccharidiques pour l'utilisation selon l'une quelconque des revendications précédentes, **caractérisés par le fait que** lesdits dérivés polysaccharidiques sont capables de stimuler la prolifération des fibroblastes au détriment des myofibroblastes.

24. Dérivés polysaccharidiques selon la revendication 23, **caractérisés par le fait que** lesdits dérivés polysaccharidiques sont utilisés pour prévenir ou traiter les processus de cicatrisation hypertrophique ou des pathologies fibrotiques des tissus conjonctifs, notamment des tissus conjonctifs dermiques et gingivaux.

25. Dérivés polysaccharidiques pour l'utilisation selon l'une quelconque des revendications précédentes, **caractérisés par le fait que** lesdits dérivés polysaccharidiques sont capables de stimuler sélectivement la prolifération des cellules médullaires à vocation mésenchymateuse au détriment d'autres sous-populations dans une population hétérogène de cellules, notamment en thérapie tissulaire ou cellulaire.

26. Composition pharmaceutique ou médicament pour utilisation comme cicatrisant des tissus conjonctifs, notamment des tissus conjonctifs dermiques et gingivaux, comprenant des dérivés polysaccharidiques de faible masse molaire et sulfatés selon l'une quelconque des revendications 1 à 25 et un excipient approprié.

27. Composition pharmaceutique ou médicament pour l'utilisation selon la revendication 26, **caractérisée en ce qu'**elle est utilisée en association avec un ou plusieurs facteurs de croissance présents au sein de la composition pharmaceutique ou présents au sein d'une composition pharmaceutique distincte, lesdits facteurs de croissance étant notamment les FGFs (Fibroblast Growth Factor), les TGFs, les BMPs (Bone Morphogenic Proteins), le CTGF (Connective tissue Growth Factor).

28. Composition pharmaceutique ou médicament pour l'utilisation selon l'une quelconque des revendications 26 à 27, **caractérisée en ce qu'**elle se présente sous la forme d'un gel, d'une crème, d'une pommade, d'une émulsion, ou d'une solution.

29. Composition pharmaceutique ou médicament pour l'utilisation selon l'une quelconque des revendications 26 à 27, **caractérisée en ce qu'**elle se présente sous une forme injectable, et dans laquelle lesdits dérivés polysaccharidiques de faible masse molaire et sulfatés présentent une masse molaire comprise entre 5 000 et 50 000 g/mol, de préférence inférieure ou égale à 25 000 g/mol, un indice de polydispersité compris entre 1,5 et 5, de préférence inférieur ou égal à 2, et un taux de substitution en groupements sulfates compris entre 10 et 45% en poids, de préférence compris entre 20 et 40% en poids inclusivement.

30. Dispositif médical pour utilisation comme cicatrisant des tissus conjonctifs, notamment des tissus conjonctifs dermiques et gingivaux, comprenant des dérivés polysaccharidiques de faible masse molaire et sulfatés selon l'une quelconque des revendications 1 à 25.

31. Dispositif médical pour l'utilisation selon la revendication 30, **caractérisé en ce qu'**il est résorbable ou non, et choisi dans le groupe consistant en des supports à libération différée, des éponges à délitement lent et des implants chirurgicaux.

## Claims

1. Low-molecular-weight sulfated polysaccharide derivatives of marine native exopolysaccharides (EPSs) excreted by mesophilic marine bacteria from a deep hydrothermal environment belonging to the *Alteromonas* genus or *Vibrio* genus for use as healing agents of connective tissues, in particular dermal and gingival connective tissues, said low-molecular-weight sulfated polysaccharide derivatives being obtainable by a process comprising the following steps:
- a step of free-radical depolymerisation of said native EPSs so as to obtain depolymerised derivatives of low molecular weight, less than or equal to 100,000 g/mol,
- a subsequent step of sulfation of the optionally lyophilized, depolymerised derivatives, comprising the addition of at least one sulfation agent in an amount sufficient to obtain sulfated polysaccharide derivatives having a degree of sulfate-group substitution of between 10% and 45% by weight relative to the total weight of the sulfated polysaccharide derivatives, said sulfation step being optionally followed by a dialysis step.

2. Low-molecular-weight sulfated polysaccharide derivatives for use according to claim 1, **characterized in that** the process further comprises a reduction step that following the free-radical depolymerisation step.

3. Low-molecular-weight sulfated polysaccharide derivatives for use according to any one of the previous claims, **characterized in that** the process further comprises a step of N-deacetylation of the polysaccharide derivatives obtained at the end of the depolymerisation step and/or at the end of the reduction step.

4. Low-molecular-weight sulfated polysaccharide derivatives for use according to any one of the previous claims, **characterized in that** the process further comprises a step of lyophilization of the polysaccharide derivatives, following the depolymerisation and/or following the reduction and/or following the N-acetylation and/or following the sulfation.

5. Low-molecular-weight sulfated polysaccharide derivatives for use according to claim 1, **characterized in that** the first free-radical depolymerisation step is carried out by addition of a solution of an oxidizing agent, preferably selected from the ions Cu⁺⁺, Fe⁺⁺, and Cr⁺⁺⁺ and the anion Cr₂O₇²⁻.

6. Low-molecular-weight sulfated polysaccharide derivatives for use according to claim 5, **characterized in that** said oxidizing agent is a solution of hydrogen peroxide, preferably having a concentration of between 0.1% and 0.5% by weight, which is added at a flow rate of between V1/1000 and V1/10 ml/minute, preferably from V1/50 to V1/500 ml/minute, V1 being the volume of the reaction mixture containing a marine exopolysaccharide (EPS) excreted by mesophilic marine bacteria from a deep hydrothermal environment, to which the solution of hydrogen peroxide is added.

7. Low-molecular-weight sulfated polysaccharide derivatives for use according to any one of the previous claims, **characterized in that** the sulfation agent(s) used in the sulfation step is/are selected from complexes of pyridine sulfate, of triethylamine sulfate, of dimethylformamide sulfate or of trimethyl sulfate.

8. Low-molecular-weight sulfated polysaccharide derivatives for use according to any one of the previous claims, **characterized in that**, for carrying out the sulfation step, the chemical sulfation agent(s) is/are added in a weight amount representing from 4 to 6 times the mass of the polysaccharide derivatives in solution and is/are added to the depolymerised EPSs which are in dry form or are in solution in a solvent, preferably an anhydrous solvent.

9. Low-molecular-weight sulfated polysaccharide derivatives for use according to any one of the previous claims, **characterized in that** they have a polydispersity index of less than 5, preferably of between 1.5 and 4, and a degree of sulfate-group substitution of between 10% and 45% by weight, and preferably between 20% and 40% by weight, inclusive.

10. Low-molecular-weight sulfated polysaccharide derivatives for use according to claim 9, **characterized in that** they have a molecular weight of less than or equal to 25 000 g/mol, a polydispersity index of less than 2, and a degree of sulfate-group substitution of between 10% and 45% by weight, and preferably between 20% and 40% by weight, inclusive.

11. Low-molecular-weight sulfated polysaccharide derivatives for use according to any one of claims 1 to 10, **characterized in that** the bacteria of the *Alteromonas* genus are of the GY785 strain.

12. Low-molecular-weight sulfated polysaccharide derivatives for use according to any one of claims 1 to 10, **characterized in that** the bacteria of the *Alteromonas* genus are selected from the strains HYD 657, HYD 708, HYD 721, HYD 1545, HYD 1644, ST 716 and MS 907.

13. Sulfated polysaccharide derivatives for use according to any one of claims 1 to 10, **characterized in that** the bacteria of the *Vibrio* genus are bacteria of the HE 800 strain.

14. Low-molecular-weight sulfated polysaccharide derivatives for use according to any one of claims 1 to 12, **characterized in that** said native exopolysaccharides (EPSs) excreted by bacteria of the *Alteromonas* genus have an oside composition comprising:
- from 20% to 70%, preferably from 30% to 60%, and more preferably from 38% to 57% by weight of neutral monosaccharides,
- from 5% to 60%, preferably from 6% to 50%, and more preferably from 8% to 42% by weight of acidic monosaccharides, and
- from 0% to 1% by weight of amino sugars.

15. Low-molecular-weight sulfated polysaccharide derivatives for use according to any one of claims 1 to 10 and 13, **characterized in that** said marine exopolysaccharides (EPSs) excreted by bacteria of the *Vibrio* genus have an oside composition comprising:
- from 0% to 5%, preferably from 0% to 1% by weight of neutral monosaccharides,
- from 20% to 50%, preferably from 25% to 40%, and more preferably from 30% to 32% by weight of acidic monosaccharides,
- from 20% to 50%, preferably from 25% to 40%, and more preferably from 30% to 35% by weight of amino sugars, and
- from 0% to 15%, preferably from 4% to 8%, and more preferably from 5% to 6% by weight of N-acetylated groups.

16. Low-molecular-weight sulfated polysaccharide derivatives for use according to any one of the previous claims, **characterized in that** said native EPSs have a protein content of from 0% to 15%, preferably from 0% to 5%, and more preferably from 0% to 1% by weight.

17. Polysaccharide derivatives for use according to any one of the previous claims, **characterized in that** said polysaccharide derivatives are capable of stimulating the proliferation of fibroblasts in two-dimensional cultures or in reconstructed connective tissues, in particular dermal and gingival connective tissues

18. Polysaccharide derivatives for use according to any one of the previous claims, **characterized in that** said polysaccharide derivatives are capable of promoting the reconstruction and the remodelling of connective tissues, in particular of dermal and gingival connective tissues.

19. Polysaccharide derivatives for use according to any one of the previous claims, **characterized in that** said polysaccharide derivatives are capable of inhibiting the secretion of pro-inflammatory cytokines by the fibroblasts of connective tissues, in particular of dermal and gingival connective tissues, and in particular of inhibiting the secretion of interleukin 1β and/or of TNF-α.

20. Polysaccharide derivatives for use according to any one of the previous claims, **characterized in that** said polysaccharide derivatives are capable of inhibiting the secretion of matrix metalloproteases by the fibroblasts of connective tissues, in particular of dermal and gingival connective tissues, and in particular of inhibiting the secretion of gelatinase A (MMP-2) and of stromelysin 1 (MMP-3).

21. Polysaccharide derivatives for use according to any one of the previous claims, **characterized in that** said polysaccharide derivatives are capable of inhibiting the conventional complement pathway.

22. Polysaccharide derivatives for use according to any one of the previous claims, **characterized in that** said polysaccharide derivatives are used for treating inflammatory pathologies, such as infectious, neoplastic or autoimmune pathologies, affecting connective tissues, in particular dermal and gingival connective tissues.

23. Polysaccharide derivatives for use according to any one of the previous claims, **characterized in that** said polysaccharide derivatives are capable of stimulating the proliferation of fibroblasts to the detriment of myofibroblasts.

24. Polysaccharide derivatives for use according to claim 23, **characterized in that** said polysaccharide derivatives are used for preventing or treating hypertrophic wound-healing processes or fibrotic pathologies of connective tissues, in particular of dermal and gingival connective tissues.

25. Polysaccharide derivatives for use according to any one of the previous claims, **characterized in that** said polysaccharide derivatives are capable of selectively stimulating the proliferation of medullary cells intended to become mesenchymal cells to the detriment of other subpopulations in a heterogeneous population of cells, in particular in tissue or cell therapy.

26. Pharmaceutical composition or medicament for use as healing agent of connective tissues, in particular dermal and gingival connective tissues, comprising low-molecular-weight sulfated polysaccharide derivatives of any one of claims 1 to 25, and an acceptable excipient.

27. Pharmaceutical composition or medicament for use according to claim 26, **characterized in that** it is used in combination with one or more growth factors present in the pharmaceutical composition or present in a different pharmaceutical composition, said growth factors being in particular FGFs (fibroblast growth factors), TGFβs, BMPS (bone morphogenic proteins) or CTGF (connective tissue growth factor).

28. Pharmaceutical composition or medicament for use according to any one of claim 26-27, **characterized in that** it is under the form of a gel, a cream, an ointment, an emulsion or a solution.

29. Pharmaceutical composition or medicament for use according to any one of claim 26-27, **characterized in that** it is in an injectable form, in which said low-molecular-weight sulfated polysaccharide derivatives have a molecular weight of between 5000 and 50 000 g/mol, preferably less than or equal to 25 000 g/mol, a polydispersity index of between 1.5 and 5, preferably less than or equal to 2, and a degree of sulfate-group substitution of between 10% and 45% by weight, and preferably between 20% and 40% by weight, inclusive.

30. Medical device for use as a healing agent of connective tissues, in particular dermal and gingival connective tissues, comprising low-molecular-weight sulfated polysaccharide derivatives according to any one of claims 1 to 25.

31. Medical device for use according to claim 30, **characterized in that** it is resorbable or nonresorbable and is selected from the group consisting of delayed-release supports, slowly disintegrating sponges, and surgical implants.

## Patentansprüche

1. Sulfatisierte Polysaccharid-Derivate mit niedriger Molmasse, die von natürlich vorkommenden, marinen Exopolysacchariden (EPS), die von mesophilen marinen Bakterien ausgeschieden werden, die aus dem tiefen hydrothermalen Milieu stammen und zur Gattung *Alteromonas* oder *Vibrio* gehören, abgeleitet sind, zur Verwendung als Mittel zur Wundheilung von Bindegewebe, insbesondere Haut- und Zahnfleisch-Bindegewebe, wobei die Derivate durch das Verfahren erhältlich sind, das die folgenden Schritte umfasst:
- einen Schritt der radikalischen Depolymerisation der natürlich vorkommenden EPS, um depolymerisierte Derivate mit niedriger Molmasse von weniger als oder gleich 100000 g/mol zu erhalten,
- einen darauffolgenden Schritt des Sulfatisierens der depolymerisierten, gegebenenfalls lyophilisierten Derivate, umfassend die Zugabe mindestens eines Sulfatisierungsmittels in einer ausreichenden Menge, um sulfatisierte Polysaccharid-Derivate zu erhalten, die einen Substitutionsgrad an Sulfatresten zwischen 10 und 45 Gew.-%, bezogen auf das Gesamtgewicht des sulfatisierten Polysaccharid-Derivats, aufweisen, wobei auf den Sulfatisierungsschritt gegebenenfalls ein Dialyseschritt folgt.

2. Sulfatisierte Polysaccharid-Derivate mit niedriger Molmasse zur Verwendung nach Anspruch 1, **dadurch gekennzeichnet, dass** das Verfahren weiter einen Reduktionsschritt nach dem radikalischen Depolymerisationsschritt umfasst.

3. Sulfatisierte Polysaccharid-Derivate mit niedriger Molmasse zur Verwendung nach einem der vorhergehenden Ansprüche, **dadurch gekennzeichnet, dass** das Verfahren weiter einen Schritt der N-Deacetylierung der Polysaccharid-Derivate umfasst, die am Ende des Depolymerisationsschrittes und/oder am Ende des Reduktionsschrittes erhalten werden.

4. Sulfatisierte Polysaccharid-Derivate mit niedriger Molmasse zur Verwendung nach einem der vorhergehenden Ansprüche, **dadurch gekennzeichnet, dass** das Verfahren weiter nach der Depolymerisation und/oder nach der Reduktion und/oder nach der N-Deacetylierung und/oder vor oder nach der Sulfatisierung einen Schritt der Lyophilisierung der Polysaccharid-Derivate umfasst.

5. Sulfatisierte Polysaccharid-Derivate mit niedriger Molmasse zur Verwendung nach Anspruch 1, **dadurch gekennzeichnet, dass** der erste Schritt der radikalischen Depolymerisation durch Zugabe einer Lösung eines Oxidationsmittels, vorzugsweise ausgewählt aus den Ionen Cu⁺⁺, Fe⁺⁺, Cr⁺⁺⁺ und dem Anion Cr₂O₇²⁻, erfolgt.

6. Sulfatisierte Polysaccharid-Derivate mit niedriger Molmasse zur Verwendung nach Anspruch 5, **dadurch gekennzeichnet, dass** das Oxidationsmittel eine Lösung von Wasserstoffperoxid, vorzugsweise in einer Konzentration zwischen 0,1 und 0,5 Gew.-%, ist, die in einer Menge zwischen V1/1000 und V1/10 ml/Minute, vorzugsweise V1/50 bis V1/500 ml/Minute, zugegeben wird, wobei V1 das Volumen des Reaktionsmediums ist, das ein marines Exopolysaccharid (EPS) enthält, das von mesophilen marinen Bakterien ausgeschieden wird, die aus dem tiefen hydrothermalen Milieu stammen, zu welchem die Wasserstoffperoxid-Lösung zugegeben wird.

7. Sulfatisierte Polysaccharid-Derivate mit niedriger Molmasse zur Verwendung nach einem der vorhergehenden Ansprüche, **dadurch gekennzeichnet, dass** das oder die für den Sulfatisierungsschritt verwendete(n) Sulfatisierungsmittel aus den Pyridinsulfat-, Triethylaminsulfat-, Dimethylformamidsulfat- oder Trimethylaminsulfat-Komplexen ausgewählt sind.

8. Sulfatisierte Polysaccharid-Derivate mit niedriger Molmasse zur Verwendung nach einem der vorhergehenden Ansprüche, **dadurch gekennzeichnet, dass** zur Durchführung des Sulfatisierungsschritts das oder die chemische(n) Sulfatisierungsmittel in einer Gewichtsmenge zugegeben werden, die das 4- bis 6-Fache der Masse der Polysaccharid-Derivate in Lösung darstellt, und den depolymerisierten EPS, die in trockener Form oder in Form einer Lösung in einem vorzugsweise wasserfreien Lösungsmittel vorliegen, zugegeben werden.

9. Sulfatisierte Polysaccharid-Derivate mit niedriger Molmasse zur Verwendung nach einem der vorhergehenden Ansprüche, **dadurch gekennzeichnet, dass** sie einen Polydispersitätsindex von weniger als 5, vorzugsweise zwischen 1,5 und 4, und einen Substitutionsgrad an Sulfatresten zwischen 10 und 45 Gew.-%, und vorzugsweise zwischen 20 und 45 Gew.-% einschließlich, aufweisen.

10. Sulfatisierte Polysaccharid-Derivate mit niedriger Molmasse zur Verwendung nach Anspruch 9, **dadurch gekennzeichnet, dass** sie eine Molmasse von weniger als oder gleich 25000 g/mol, einen Polydispersitätsindex von weniger als 2 und einen Substitutionsgrad an Sulfatresten zwischen 10 und 45 Gew.-%, und vorzugsweise zwischen 20 und 40 Gew.-% einschließlich, aufweisen.

11. Sulfatisierte Polysaccharid-Derivate mit niedriger Molmasse zur Verwendung nach einem der Ansprüche 1 bis 10, **dadurch gekennzeichnet, dass** die Bakterien der Gattung *Alteromonas* vom Stamm GY785 sind.

12. Sulfatisierte Polysaccharid-Derivate mit niedriger Molmasse zur Verwendung nach einem der Ansprüche 1 bis 10, **dadurch gekennzeichnet, dass** die Bakterien der Gattung *Alteromonas* aus den Stämmen HYD 657, HYD 708, HYD 721, HYD 1545, HYD 1644, ST 716 und MS 907 ausgewählt sind.

13. Sulfatisierte Polysaccharid-Derivate mit niedriger Molmasse zur Verwendung nach einem der Ansprüche 1 bis 10, **dadurch gekennzeichnet, dass** die Bakterien der Gattung *Vibrio* Bakterien vom Stamm HE 800 sind.

14. Sulfatisierte Polysaccharid-Derivate mit niedriger Molmasse zur Verwendung nach einem der Ansprüche 1 bis 12, **dadurch gekennzeichnet, dass** die natürlich vorkommenden, von Bakterien der Gattung *Alteromonas* ausgeschiedenen Exopolysaccharide (EPS) eine Monosaccharidzusammensetzung aufweisen, welche umfasst:
- 20 bis 70 Gew.-%, vorzugsweise 30 bis 60 Gew.-%, und stärker bevorzugt 38 bis 57 Gew.-% neutrale Monosaccharide,
- 5 bis 60 Gew.-%, vorzugsweise 6 bis 50 Gew.-%, und stärker bevorzugt 8 bis 42 Gew.-% Zuckersäuren und
- 0 bis 1 Gew.-% Aminozucker.

15. Sulfatisierte Polysaccharid-Derivate mit niedriger Molmasse zur Verwendung nach einem der Ansprüche 1 bis 10 und 13, **dadurch gekennzeichnet, dass** die von Bakterien der Gattung *Tlibrio* ausgeschiedenen marinen Exopolysaccharide (EPS) eine Monosaccharidzusammensetzung aufweisen, welche umfasst:
- 0 bis 5 Gew.-%, vorzugsweise 0 bis 1 Gew.-%, neutrale Monosaccharide,
- 20 bis 50 Gew.-%, vorzugsweise 25 bis 40 Gew.-%, und stärker bevorzugt 30 bis 32 Gew.-% Zuckersäuren,
- 20 bis 50 Gew.-%, vorzugsweise 25 bis 40 Gew.-%, und stärker bevorzugt 30 bis 35 Gew.-% Aminozucker und
- 0 bis 15 Gew.-%, vorzugsweise 4 bis 8 Gew.-%, und stärker bevorzugt 5 bis 6 Gew.-% N-acetylierte Reste.

16. Sulfatisierte Polysaccharid-Derivate mit niedriger Molmasse zur Verwendung nach einem der vorhergehenden Ansprüche, **dadurch gekennzeichnet, dass** die natürlich vorkommenden EPS einen Gehalt von 0 bis 15 Gew.-%, vorzgusweise 0 bis 5 Gew.-%, und stärker bevorzugt 0 bis 1 Gew.-% an Proteinen aufweisen.

17. Polysaccharid-Derivate zur Verwendung nach einem der vorhergehenden Ansprüche, **dadurch gekennzeichnet, dass** die Polysaccharid-Derivate in der Lage sind, die Proliferation von Fibroblasten in zweidimensionalen Kulturen oder im Inneren von rekonstruiertem Bindegewebe, insbesondere Haut- und Zahnfleisch-Bindegewebe, zu stimulieren.

18. Polysaccharid-Derivate zur Verwendung nach einem der vorhergehenden Ansprüche, **dadurch gekennzeichnet, dass** die Polysaccharid-Derivate es ermöglichen, die Rekonstruktion und Remodellierung von Bindegewebe, insbesondere Haut- und Zahnfleisch-Bindegewebe, zu begünstigen.

19. Polysaccharid-Derivate zur Verwendung nach einem der vorhergehenden Ansprüche, **dadurch gekennzeichnet, dass** die Polysaccharid-Derivate in der Lage sind, die Sekretion von entzündungsfördernden Cytokinen durch die Fibroblasten des Bindegewebes, insbesondere des Haut- und Zahnfleisch-Bindegewebes, zu hemmen, und insbesondere die Sekretion von Interleukin-1β und/oder TNF-α zu hemmen.

20. Polysaccharid-Derivate zur Verwendung nach einem der vorhergehenden Ansprüche, **dadurch gekennzeichnet, dass** die Polysaccharid-Derivate in der Lage sind, die Sekretion von Matrix-Metallproteasen durch die Fibroblasten des Bindegewebes, insbesondere des Haut- und Zahnfleisch-Bindegewebes, zu hemmen und insbesondere die Gelatinase A (MMP-2)- und die Stromelysin 1 (MMP-3)-Sekretion zu hemmen.

21. Polysaccharid-Derivate zur Verwendung nach einem der vorhergehenden Ansprüche, **dadurch gekennzeichnet, dass** die Polysaccharid-Derivate in der Lage sind, den klassischen Komplementpfad zu hemmen.

22. Polysaccharid-Derivate zur Verwendung nach einem der vorhergehenden Ansprüche, **dadurch gekennzeichnet, dass** die Polysaccharid-Derivate zur Behandlung entzündlicher Erkrankungen, wie infektiöser, neoplastischer oder Autoimmun-Erkrankungen, die das Bindegewebe, insbesondere das Haut- oder Zahnfleisch-Bindegewebe, betreffen, verwendet werden.

23. Polysaccharid-Derivate zur Verwendung nach einem der vorhergehenden Ansprüche, **dadurch gekennzeichnet, dass** die Polysaccharid-Derivate in der Lage sind, die Proliferation der Fibroblasten zu Lasten der Myofibroblasten zu stimulieren.

24. Polysaccharid-Derivate zur Verwendung nach Anspruch 23, **dadurch gekennzeichnet, dass** die Polysaccharid-Derivate zur Prävention oder Behandlung hypertropher Wundheilungsprozesse oder fibrotischer Erkrankungen des Bindegewebes, insbesondere des Haut- und Zahnfleisch-Bindegewebes, verwendet werden.

25. Polysaccharid-Derivate zur Verwendung nach einem der vorhergehenden Ansprüche, **dadurch kennzeichnet, dass** die Polysaccharid-Derivate in der Lage sind, selektiv die Proliferation der medullären Zellen mesenchymalen Ursprungs zu Lasten anderer Subpopulationen in einer heterogenen Zellpopulation, insbesondere bei der Gewebe- oder Zelltherapie, zu stimulieren.

26. Arzneimittel oder Medikament zur Verwendung als die Wundheilung von Bindegewebe, insbesondere Haut- und Zahnfleisch-Bindegewebe, förderndes Mittel, umfassend die sulfatisierten Polysaccharid-Derivate mit niedriger Molmasse nach einem der Ansprüche 1 bis 25 und einen geeigneten Exzipienten.

27. Arzneimittel oder Medikament zur Verwendung nach Anspruch 26, **dadurch gekennzeichnet, dass** es in Verbindung mit einem oder mehreren Wachstumsfaktoren verwendet wird, die in dem Arzneimittel oder in einem separaten Arzneimittel vorliegen, wobei die Wachstumsfaktoren insbesondere FGF (Fibroblast-Wachstumsfaktor), TGF, BMP (knochenmorphogene Proteine), CTGF (Bindegewebs-Wachstumsfaktor) sind.

28. Arzneimittel oder Medikament zur Verwendung nach einem der Ansprüche 26 bis 27, **dadurch gekennzeichnet, dass** es in Form eines Gels, einer Creme, einer Salbe, einer Emulsion oder einer Lösung vorliegt.

29. Arzneimittel oder Medikament zur Verwendung nach einem der Ansprüche 26 bis 27, **dadurch gekennzeichnet, dass** es in einer injizierbaren Form vorliegt, und wobei die sulfatisierten Polysaccharid-Derivate mit niedriger Molmasse eine Molmasse zwischen 5000 und 50000 g/mol, vorzugsweise weniger als oder gleich 25000 g/mol, einen Polydispersitätsindex zwischen 1,5 und 5, vorzugsweise weniger als oder gleich 2, und einen Substitutionsgrad an Sulfatresten zwischen 10 und 45 Gew.%, vorzugsweise zwischen 20 und 40 Gew.-% einschließlich, aufweisen.

30. Medizinischer Gegenstand zur Verwendung als die Wundheilung von Bindegewebe, insbesondere Haut- und Zahnfleisch-Bindegewebe, förderndes Mittel, umfassend die sulfatisierten Polysaccharid-Derivate mit niedriger Molmasse nach einem der Ansprüche 1 bis 25.

31. Medizinischer Gegenstand zur Verwendung nach Anspruch 30, **dadurch gekennzeichnet, dass** er resorbierbar oder nicht resorbierbar ist und aus der Gruppe bestehend aus Trägern mit verzögerter Freisetzung, Schwämmen mit langsamer Abgabe und chirurgischen Implantaten ausgewählt ist.
